# EUROPEAN PATENT APPLICATION

(11) **EP 2 512 143 A1**
(43) Date of publication of application: **17.10.2012**
(21) Application number: 11843752.4
(22) Date of filing: 01.11.2011
(51) Int. Cl.: H04N 13/00

(54) **IMAGE DATA TRANSMISSION DEVICE, IMAGE DATA TRANSMISSION METHOD, IMAGE DATA RECEPTION DEVICE, AND IMAGE DATA RECEPTION METHOD**

(30) Priority: 22.11.2010 JP 2010260193
(71) Applicant: Sony Corporation, Tokyo 108-0075 (JP)
(72) Inventor: TSUKAGOSHI, Ikuo, Tokyo 108-0075 (JP)
(74) Representative: Beder, Jens
(86) International application number: PCT/JP2011/075135
(87) International publication number: WO 2012/070364

(57) **Abstract**

[Object] To enable a reception side (3DTV) to correctly generate left-eye and right-eye display image data by suitably performing cropping processing using cropping information.

[Solution] "Stereo_Video_Cropping SEI" including 3D display cropping information is inserted, together with 2D display cropping information, into a header of a video data stream. A 3DTV at a reception side is able to perform image data cropping processing on the basis of this cropping information. Flag information "stereo_video_cropping_SEI_Not_present_flag" indicating whether or not 3D display cropping information is present is inserted into a higher layer of a data stream. The 3DTV at the reception side is able to identify the presence or absence of 3D display cropping information without analyzing the header of the video data stream.

## Description

### Technical Field

The present invention relates to image data transmitting apparatuses, image data transmitting methods, image data receiving apparatuses, and image data receiving methods. Particularly, the present invention relates to an image data transmitting apparatus, etc. used in an image transmitting/receiving system in which cropping information is transmitted together with three-dimensional image data from a transmission side and image data cropping processing is performed by using this cropping information at a reception side.

### Background Art

For example, in PTL 1, a transmission method for transmitting three-dimensional image data by using television broadcast waves has been proposed. In this case, three-dimensional image data including left-eye image data and right-eye image data is transmitted, and a television receiver performs three-dimensional image display utilizing binocular parallax.

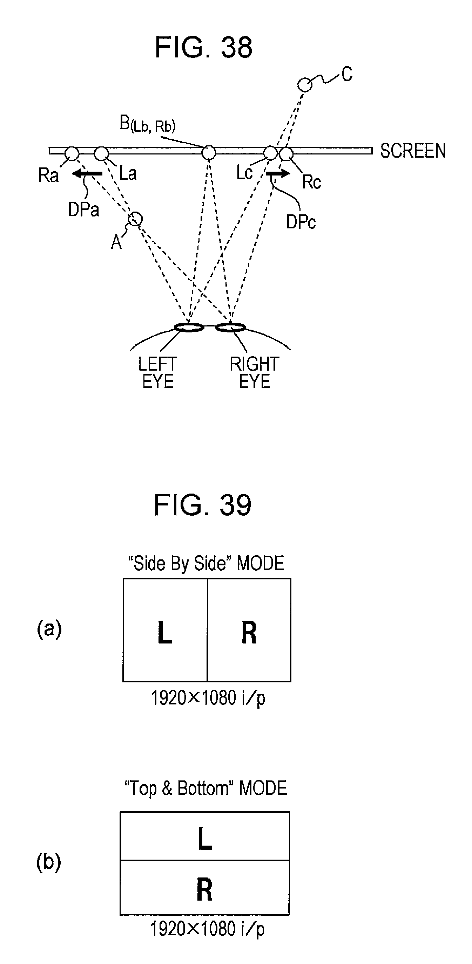

Fig. 38 illustrates, in three-dimensional image display utilizing binocular parallax, the relationship between the display positions of a left image and a right image of an object on a screen and the playback position of the three-dimensional image (3D image) of the left image and the right image. For example, concerning object A for which a left image La thereof is displayed on the right side on the screen and for which a right image Ra thereof is displayed on the left side on the screen, as shown in the drawing, the line of sight of the left eye and the line of sight of the right eye cross each other in front of the screen surface, and thus, the playback position of the three-dimensional image is in front of the screen surface. DPa is a horizontal-direction parallax vector concerning the object A.

Concerning object B for which a left image Lb thereof and a right image Rb thereof are displayed at the same position on the screen, as shown in the drawing, the line of sight of the left eye and the line of sight of the right eye cross each other on the screen surface, and thus, the playback position of the three-dimensional image is on the screen surface. Concerning object C for which a left image Lc thereof is displayed on the left side and for which a right image Rc is displayed on the right side, as shown in the drawing, the line of sight of the left eye and the line of sight of the right eye cross each other behind the screen surface, and thus, the playback position of the three-dimensional image is behind the screen surface. DPc is a horizontal-direction parallax vector for the object C.

In the related art, transmission formats of three-dimensional image data include a side-by-side mode, a top-and-bottom mode, etc. Part (a) of Fig. 39 illustrates the side-by-side mode, while part (b) of Fig. 39 illustrates the top-and-bottom mode. Here, part (a) and part (b) of Fig. 39 illustrate the modes when a 1920 x 1080-pixel format is used.

In the side-by-side mode, as shown in part (a) of Fig. 39, in a first half in the horizontal direction, pixel data of left-eye image data is transmitted, and in a second half in the horizontal direction, pixel data of right-eye image data is transmitted. In this mode, the pixel data of the left-eye image data and that of the right-eye image data in the horizontal direction are each scaled down by 1/2, and the horizontal resolution of the left-eye image data and that of the right-eye image data are each halved with respect to an original signal.

In the top-and-bottom mode, as shown in part (b) of Fig. 39, in a first half in the vertical direction, line data of left-eye image data is transmitted, and in a second half in the vertical direction, line data of right-eye image data is transmitted. In this mode, the lines of the left-eye image data and those of the right-eye image data are each scaled down by 1/2, and the vertical resolution of the left-eye image data and that of the right-eye image data are each halved with respect to the original signal.

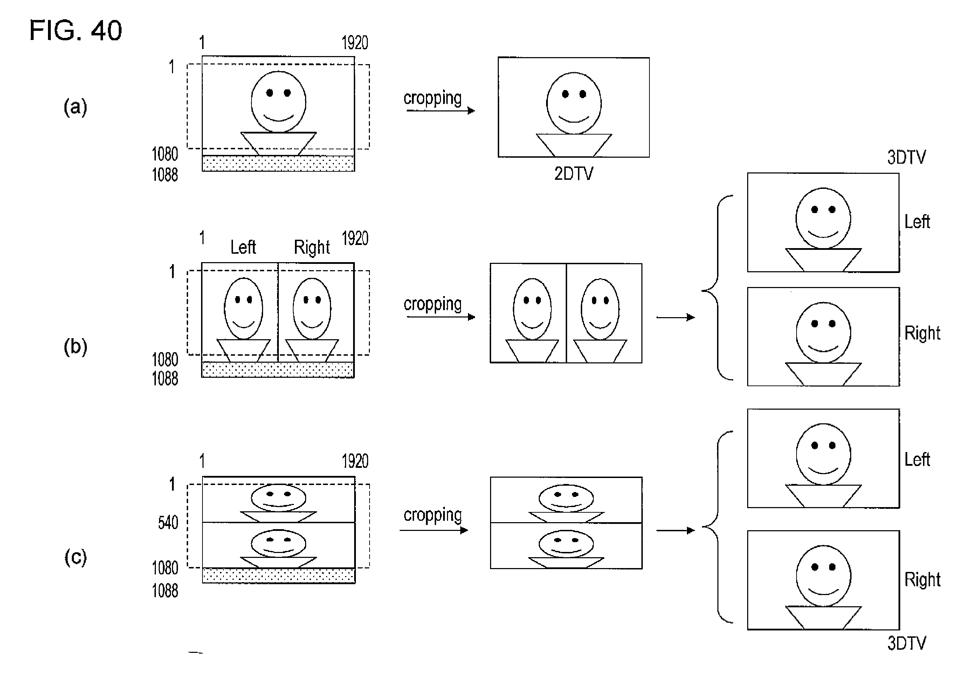

Display image data generating processing performed by a reception side will be briefly described below. Part (a) of Fig. 40 schematically illustrates processing for two-dimensional image data having a 1920 x 1080-pixel format. In this case, in the transmission side, in order to perform encoding in units of 16x16 blocks, eight lines formed of blank data are added to the 1920 x 1080-pixel format, resulting in 1920-pixel x 1088-line image data, which is then encoded.

Accordingly, in the reception side, the 1920-pixel x 1088-line image data is obtained. However, since eight lines are formed of blank data, 1920-pixel x 1080-line image data, which contains actual image data, is cropped on the basis of cropping information contained in a video data stream, thereby generating display image data for a two-dimensional television receiver (hereinafter may be referred to as a "2DTV" as appropriate).

Part (b) of Fig. 40 schematically illustrates processing for side-by-side mode three-dimensional image data having a 1920 x 1080-pixel format. In this case, too, in the transmission side, in order to perform encoding in units of 16x16 blocks, eight lines formed of blank data are added to the 1920 x 1080-pixel format, resulting in 1920-pixel x 1088-line image data, which is then encoded.

Accordingly, in the reception side, after decoding, the 1920-pixel x 1088-line image data is obtained. However, since eight lines are formed of blank data, 1920-pixel x 1080-line image data, which contains actual image data, is cropped on the basis of cropping information contained in a video data stream. Then, the image data is divided into left and right frames, which are then each subjected to scaling processing, thereby generating left-eye display image data and right-eye display image data for a three-dimensional television receiver (hereinafter may be referred to as a "3DTV" as appropriate).

Part (c) of Fig. 40 schematically illustrates processing for top-and-bottom mode three-dimensional image data having a 1920 x 1080-pixel format. In this case, too, in the transmission side, in order to perform encoding in units of 16x16 blocks, eight lines formed of blank data are added to the 1920 x 1080-pixel format, resulting in 1920-pixel x 1088-line image data, which is then encoded.

Accordingly, in the reception side, after decoding, the 1920-pixel x 1088-line image data is obtained. However, since eight lines are formed of blank data, 1920-pixel x 1080-line image data, which contains actual image data, is cropped on the basis of cropping information contained in a video data stream. Then, the image data is divided into top and bottom frames, which are then each subjected to scaling processing, thereby generating left-eye display image data and right-eye display image data for a 3DTV.

### Citation List

### Patent Literature

PTL 1: Japanese Unexamined Patent Application Publication No. 2005-6114

### Summary of Invention

### Technical Problem

In the 2DTV, when 2DTV display data is generated by cropping 1920-pixel x 1080-line image data from three-dimensional image data employing the above-described side-by-side or top-and-bottom mode, similar images are arranged side by side or above and below, thereby making the image display look unnatural.

Thus, in order to avoid image display from looking unnatural in the 2DTV, cropping information, contained in a video data stream, for cropping only one of left-eye image data and right-eye image data, for example, only the left-eye image data, may be used. Processing performed by a 2DTV and a 3DTV using this technique is as follows.

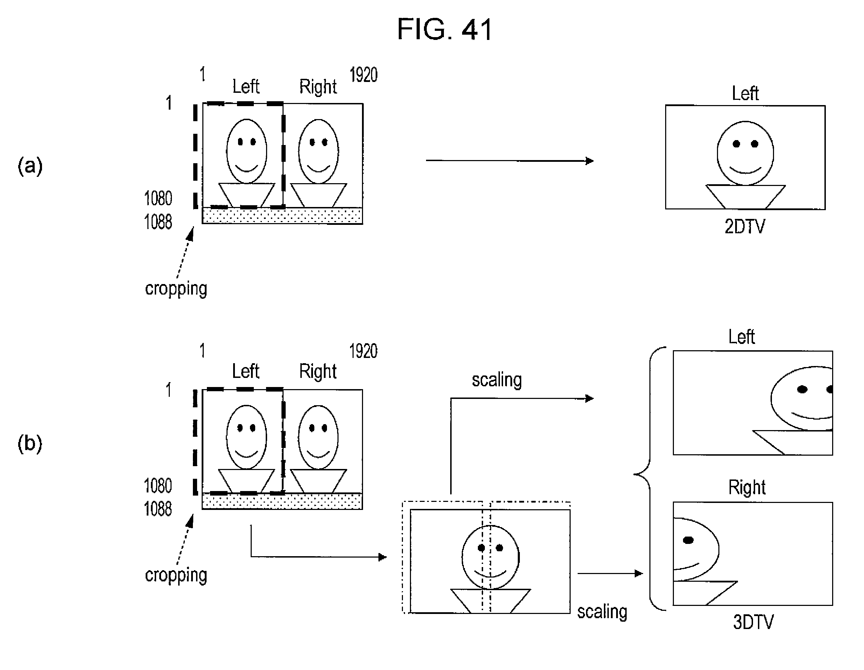

Part (a) of Fig. 41 schematically illustrates processing performed by a 2DTV on side-by-side mode three-dimensional image data having a 1920 x 1080-pixel format. In the 2DTV, after decoding, 1920-pixel x 1088-line image data is obtained. However, eight lines are formed of blank data. In this case, among the 1920-pixel x 1080-line image data, which contains actual image data, 960-pixel x 1080-line left-eye image data is cropped on the basis of cropping information. Then, the left-eye image data is subjected to scaling processing, thereby generating 2DTV display image data. In this case, the image display looks natural.

On the other hand, part (b) of Fig. 41 schematically illustrates processing performed by a 3DTV on side-by-side mode three-dimensional image data having a 1920 x 1080-pixel format. In the 3DTV, too, after decoding, 1920-pixel x 1088-line image data is obtained. However, eight lines are formed of blank data. In this case, among the 1920-pixel x 1080-line image data, which contains actual image data, 960-pixel x 1080-line left-eye image data is cropped on the basis of cropping information.

Then, the left-eye image data is subjected to scaling processing, thereby generating 1920-pixel x 1088-line image data. This image data is the same as the above-described 2DTV display image data. In the 3DTV, since the side-by-side mode is employed, the image data is further divided into left and right frames, which are then each subjected to scaling processing, thereby generating 3DTV left-eye display image data and right-eye display image data. In this case, the left-eye image and the right-eye image are merely one part and the other part divided from one image in the left and right direction. Thus, three-dimensional display (3D display) cannot be correctly performed.

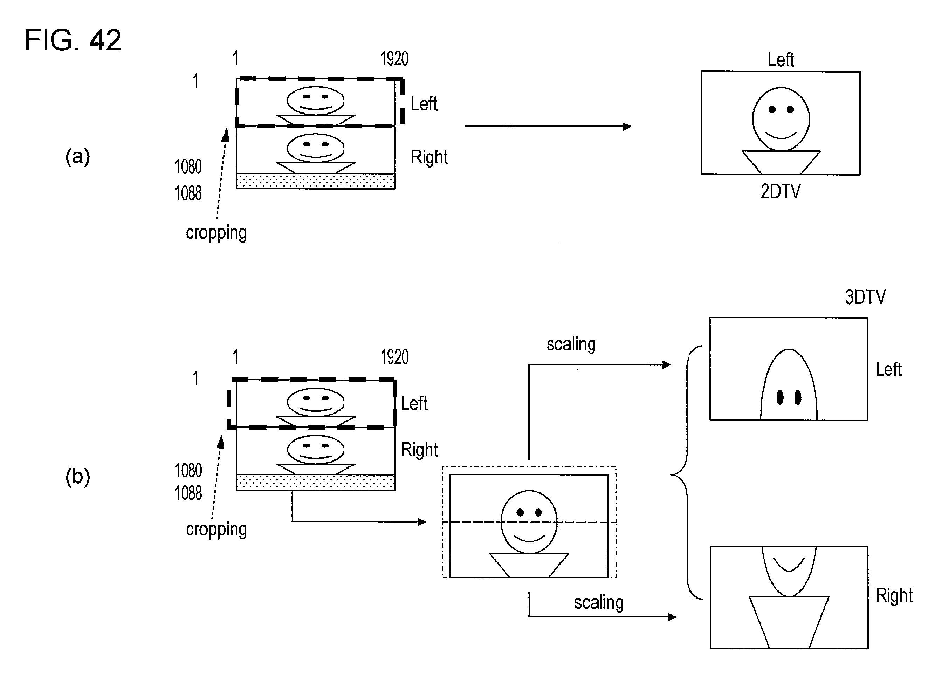

Part (a) of Fig. 42 schematically illustrates processing performed by a 2DTV on top-and-bottom mode 3D image data having a 1920 x 1080-pixel format. In the 2DTV, after decoding, 1920-pixel x 1088-line image data is obtained. However, eight lines are formed of blank data. In this case, among the 1920-pixel x 1080-line image data, which contains actual image data, 1920-pixel x 540-line left-eye image data is cropped on the basis of cropping information. Then, the left-eye image data is subjected to scaling processing, thereby generating 2DTV display image data. In this case, correct two-dimensional display (2D display) can be performed.

On the other hand, part (b) of Fig. 42 schematically illustrates processing performed by a 3DTV on top-and-bottom mode three-dimensional image data having a 1920 x 1080-pixel format. In the 3DTV, too, after decoding, 1920-pixel x 1088-line image data is obtained. However, eight lines are formed of blank data. In this case, among the 1920-pixel x 1080-line image data, which contains actual image data, 1920-pixel x 540-line left-eye image data is cropped on the basis of cropping information.

Then, the left-eye image data is subjected to scaling processing, thereby generating 1920-pixel x 1088-line image data. This image data is the same as the above-described 2DTV display image data. In the 3DTV, since the top-and-bottom mode is employed, the image data is further divided into top and bottom frames, which are then each subjected to scaling processing, thereby generating 3DTV left-eye display image data and right-eye display image data. In this case, the left-eye image and the right-eye image are merely one part and the other part divided from one image in the top and bottom direction. Thus, three-dimensional display (3D display) cannot be correctly performed.

An object of this invention is to correctly generate display image data by suitably performing cropping processing using cropping information in a reception side. Solution to Problem

An aspect of this invention is an image data transmitting apparatus including: an image data output unit that outputs three-dimensional image data including left-eye image data and right-eye image data; and a transmitter that transmits a multiplexed data stream including a data stream, the data stream including the three-dimensional image data output from the image data output unit, first cropping information used for two-dimensional display and second cropping information used for three-dimensional display being inserted into a header of the data stream.

According to this invention, the image data output unit outputs, for example, side-by-side or top-and-bottom three-dimensional image data including left-eye image data and right-eye image data. Then, the transmitter transmits a multiplexed data stream including a data stream (video elementary stream) having this three-dimensional image data. In this case, the second cropping information used for three-dimensional display, as well as the first cropping information used for two-dimensional display, is inserted into the header of the data stream.

In this manner, according to this invention, the second cropping information used for three-dimensional display, as well as the first cropping information used for two-dimensional display, is inserted into the header of the data stream, and a 3DTV at a reception side is able to perform image data cropping processing on the basis of this cropping information. Thus, the 3DTV at the reception side is able to correctly generate left-eye and right-eye display image data, thereby enabling correct three-dimensional display.

According to this invention, for example, information indicating until when a cropping state represented by the second cropping information continues may be added to the second cropping information. In this case, the 3DTV at the reception side is able to easily identify until when the cropping state represented by the second cropping information continues. For example, this information indicates that the cropping state continues until the next cropping information appears, or that the cropping state continues only during the current picture.

Additionally, according to this invention, the transmitter may insert, into a higher layer of the data stream, flag information indicating whether the second cropping information is contained in the header of the data stream. In this case, the flag information may be inserted under the program map table. For example, the flag information may be inserted as a program descriptor of the program map table. Alternatively, the flag information may be inserted under a video elementary loop of the program map table. In this case, the 3DTV at the reception side is able to identify the presence or absence of the second cropping information without analyzing the header of the video data stream.

Additionally, another aspect of this invention is an image data receiving apparatus including: a receiver that receives a multiplexed data stream including a data stream, the data stream including three-dimensional image data having left-eye image data and right-eye image data, first cropping information used for two-dimensional display and second cropping information used for three-dimensional display being inserted into a header of the data stream; and an image data processor that generates left-eye and right-eye display image data on the basis of the three-dimensional image data obtained from the multiplexed data stream received by the receiver. The image data processor performs image data cropping processing on the basis of the second cropping information contained in the header of the data stream.

According to this invention, the receiver receives a multiplexed data stream including a data stream. This data stream includes, for example, side-by-side or top-and-bottom three-dimensional image data including left-eye image data and right-eye image data. Additionally, the second cropping information used for three-dimensional display, as well as the first cropping information used for two-dimensional display, is inserted into this data stream.

The image data processor generates left-eye display image data and right-eye display image data on the basis of the three-dimensional image data obtained from the multiplexed data stream received by the receiver. In this case, the image data processor performs image data cropping processing on the basis of the second cropping information used for three-dimensional display contained in the data stream.

In this manner, according to this invention, image data cropping processing is performed on the basis of the second cropping information used for three-dimensional display inserted into the header of the data stream. Thus, left-eye and right-eye display image data are correctly generated, thereby enabling correct three-dimensional display.

Additionally, another aspect of this invention is an image data receiving apparatus including: a receiver that receives a multiplexed data stream including a data stream, the data stream including a three-dimensional image data having left-eye image data and right-eye image data, cropping information used for two-dimensional display being inserted into a header of the data stream; and an image data processor that generates left-eye and right-eye display image data on the basis of the three-dimensional image data obtained from the multiplexed data stream received by the receiver. The image data processor converts the cropping information used for two-dimensional display contained in the header of the data stream into cropping information used for three-dimensional display and performs image data cropping processing on the basis of the cropping information used for three-dimensional display.

According to this invention, the receiver receives a multiplexed data stream including a data stream. This data stream includes, for example, side-by-side or top-and-bottom three-dimensional image data including left-eye image data and right-eye image data. Additionally, the cropping information used for two-dimensional display is inserted into the header of this data stream. The image data processor generates left-eye display image data and right-eye display image data on the basis of the three-dimensional image data obtained from the multiplexed data stream received by the receiver.

In this case, the image data processor converts the cropping information used for two-dimensional display contained in the data stream into cropping information used for three-dimensional display and performs image data cropping processing on the basis of the cropping information used for three-dimensional display. Thus, left-eye and right-eye display image data are correctly generated, thereby enabling correct three-dimensional display.

Additionally, another aspect of this invention is an image data receiving apparatus including: a receiver that receives a multiplexed data stream including a data stream, the data stream including three-dimensional image data having left-eye image data and right-eye image data, cropping information used for two-dimensional display being inserted into a header of the data stream; and an image data processor that generates left-eye and right-eye display image data on the basis of the three-dimensional image data obtained from the multiplexed data stream received by the receiver. The image data processor performs image data cropping processing on the basis of the cropping information used for two-dimensional display so as to generate one of left-eye and right-eye display image data, and the image data processor generates the other one of the left-eye and the right-eye display image data on the basis of image data that remains after performing the image data cropping processing on the basis of the cropping information used for two-dimensional display.

According to this invention, the receiver receives a multiplexed data stream including a data stream. This data stream includes, for example, side-by-side or top-and-bottom three-dimensional image data including left-eye image data and right-eye image data. Additionally, the cropping information used for two-dimensional display is inserted into the header of this data stream. The image data processor generates left-eye and right-eye display image data on the basis of the three-dimensional image data obtained from the multiplexed data stream received by the receiver.

In this case, the image data processor performs image data cropping processing on the basis of the cropping information used for 2D display contained in the data stream so as to generate one of left-eye and right-eye display image data. The image data processor also generates the other one of the left-eye and the right-eye display image data on the basis of image data that remains after performing the image data cropping processing on the basis of the cropping information used for two-dimensional display. Thus, left-eye and right-eye display image data are correctly generated, thereby enabling correct three-dimensional display.

Additionally, another still aspect of this invention is an image data transmitting apparatus including: an image data output unit that outputs three-dimensional image data including left-eye image data and right-eye image data; and a transmitter that transmits a multiplexed data stream including a data stream, the data stream including the three-dimensional image data output from the image data output unit, cropping information being inserted into a header of the data stream. The transmitter inserts, into the header of the data stream or a higher layer of the data stream, identification information for identifying whether the cropping information is cropping information used for a two-dimensional image or cropping information used for a three-dimensional image.

According to this invention, the image data output unit outputs, for example, side-by-side or top-and-bottom three-dimensional image data including left-eye image data and right-eye image data. Then, the transmitter transmits a multiplexed data stream including a data stream (video elementary stream) having this three-dimensional image data. In this case, the cropping information is inserted into the header of the data stream. This cropping information is cropping information used for a two-dimensional image or for a three-dimensional image.

The transmitter inserts, into the header of the data stream or a higher layer of the data stream, identification information for identifying whether the cropping information is cropping information used for a two-dimensional image or for a three-dimensional image. In this case, the identification information for identifying that the cropping information is cropping information used for a three-dimensional image includes information indicating whether the cropping information is cropping information for left-eye image data or cropping information for right-eye image data. For example, the identification information may be inserted under the program map table. For example, the identification information may be inserted as a program descriptor of the program map table. Alternatively, the identification information may be inserted under a video elementary loop of the program map table.

In this manner, according to this invention, identification information for identifying whether the cropping information is cropping information used for a two-dimensional image or cropping information used for a three-dimensional image is inserted into the header of the data stream or a higher layer of the data stream. Thus, a 3DTV at a reception side is able to easily identify whether the cropping information contained in the header of the data stream is for a two-dimensional image or a three-dimensional image, thereby enabling suitable processing by using this cropping information.

Then, if the cropping information is for a two-dimensional image, the 3DTV at the reception side performs image data cropping information on the basis of the cropping information so as to generate left-eye and right-eye display image data. On the other hand, if the cropping information is for a three-dimensional image, for example, the 3DTV at the reception side converts that cropping information into cropping information used for a two-dimensional image. Then, image data cropping information is performed on the basis of this two-dimensional cropping information so as to generate left-eye and right-eye display image data. Thus, left-eye and right-eye display image data are correctly generated, thereby enabling correct three-dimensional display.

According to this invention, for example, the identification information may be inserted into the header of the data stream, and information indicating until when a cropping state represented by the cropping information continues may be added to the identification information. In this case, the 3DTV at the reception side is able to easily identify until when the cropping state represented by the cropping information continues. For example, this information indicates that the cropping state continues until the next cropping information appears, or that the cropping state continues only during the current picture.

Additionally, another aspect of this invention is an image data receiving apparatus including: a receiver that receives a multiplexed data stream including a data stream, the data stream including three-dimensional image data having left-eye image data and right-eye image data, cropping information used for three-dimensional display and transmission format information for the three-dimensional image data being inserted into a header of the data stream; and an image data processor that generates two-dimensional display image data on the basis of the three-dimensional image data obtained from the multiplexed data stream received by the receiver. The image data processor performs image data cropping processing and scaling processing on the basis of the cropping information used for three-dimensional display and the transmission format information for the three-dimensional image data contained in the header of the data stream.

According to this invention, the receiver receives a multiplexed data stream including a data stream. This data stream includes, for example, side-by-side or top-and-bottom three-dimensional image data including left-eye image data and right-eye image data. Additionally, the cropping information used for three-dimensional display and the transmission format information for the three-dimensional image data are inserted into the header of this data stream. The image data processor generates two-dimensional display image data on the basis of the three-dimensional image data obtained from the multiplexed data stream received by the receiver.

In this case, the image data processor performs image data cropping processing and scaling processing on the basis of the cropping information used for three-dimensional display and the transmission format information for the three-dimensional image data contained in the header of the data stream. More specifically, on the basis of the cropping information and the transmission format information, part of three-dimensional image data, for example, left-eye image data, is cropped, and the cropped image data is subjected to scaling processing in the direction corresponding to the transmission format. For example, if the transmission format is the side-by-side mode, the cropped image data is scaled in the horizontal direction. If the transmission format is the top-and-bottom mode, the cropped image data is scaled in the vertical direction. Thus, two-dimensional display image data is correctly generated, thereby enabling correct two-dimensional display.

### Advantageous Effects of Invention

According to this invention, a reception side is able to correctly generate left-eye and right-eye display image data or two-dimensional display image data by suitably performing image data cropping processing, thereby enabling correct three-dimensional display.

### Brief Description of Drawings

[Fig. 1] Fig. 1 is a block diagram illustrating an example of the configuration of an image transmitting/receiving system in accordance with a first embodiment of this invention.
[Fig. 2] Fig. 2 is a block diagram illustrating an example of the configuration of a transmission data generator in a broadcasting station forming the image transmitting/receiving system.
[Fig. 3] Fig. 3 illustrates examples of the data structure of access units in a video data stream.
[Fig. 4] Fig. 4 illustrates the structure of cropping information defined in a SPS (Sequence Parameter Set) of an access unit.
[Fig. 5] Fig. 5 illustrates an example of the structure and the principal data definition contents of "Stereo_Video_Cropping SEI".
[Fig. 6] Fig. 6 illustrates an example of the configuration of a transport stream TS when flag information is inserted under a video elementary loop of a program map table.
[Fig. 7] Fig. 7 illustrates an example of the structure and the principal data description contents of "AVC_video_descriptor".
[Fig. 8] Fig. 8 illustrates an example of the configuration of a transport stream TS when flag information is inserted as a program descriptor of a program map table.
[Fig. 9] Fig. 9 illustrates an example of the structure and the principal data description contents of "Stereo_Video_cropping_descriptor".
[Fig. 10] Fig. 10 is a block diagram illustrating an example of the configuration of a receiver (3DTV) forming the image transmitting/receiving system.
[Fig. 11] Fig. 11 illustrates processing (side-by-side mode) performed by a 3D signal processor, etc. of a receiver in the first embodiment.
[Fig. 12] Fig. 12 illustrates processing (top-and-bottom mode) performed by a 3D signal processor, etc. of a receiver in the first embodiment.
[Fig. 13] Fig. 13 is a block diagram illustrating an example of the configuration of a receiver (2DTV) forming the image transmitting/receiving system.
[Fig. 14] Fig. 14 is a block diagram illustrating an example of the configuration of an image transmitting/receiving system in accordance with a second embodiment of this invention.
[Fig. 15] Fig. 15 is a block diagram illustrating an example of a transmission data generator in a broadcasting station forming the image transmitting/receiving system.
[Fig. 16] Fig. 16 illustrates examples of the data structure of access units in a video data stream.
[Fig. 17] Fig. 17 is a block diagram illustrating an example of the configuration of a receiver (3DTV) forming the image transmitting/receiving system.
[Fig. 18] Fig. 18 illustrates processing (side-by-side mode) performed by a 3D signal processor, etc. of a receiver in the second embodiment.
[Fig. 19] Fig. 19 illustrates processing (top-and-bottom mode) performed by a 3D signal processor, etc. of a receiver in the second embodiment.
[Fig. 20] Fig. 20 is a block diagram illustrating an example of the configuration of an image transmitting/receiving system in accordance with a third embodiment of this invention.
[Fig. 21] Fig. 21 is a block diagram illustrating an example of the configuration of a receiver (3DTV) forming the image transmitting/receiving system.
[Fig. 22] Fig. 22 illustrates processing (side-by-side mode) performed by a 3D signal processor, etc. of a receiver in the third embodiment.
[Fig. 23] Fig. 23 illustrates processing (top-and-bottom mode) performed by a 3D signal processor, etc. of a receiver in the third embodiment.
[Fig. 24] Fig. 24 is a block diagram illustrating an example of the configuration of an image transmitting/receiving system in accordance with a fourth embodiment of this invention.
[Fig. 25] Fig. 25 is a block diagram illustrating an example of a transmission data generator in a broadcasting station forming the image transmitting/receiving system.
[Fig. 26] Fig. 26 illustrates examples of the data structure of access units in a video data stream.
[Fig. 27] Fig. 27 illustrates an example of the structure of "Cropping_Rectangle_Target SEI".
[Fig. 28] Fig. 28 illustrates an example of the principal data description contents of "Cropping_Rectangle_Target SEI".
[Fig. 29] Fig. 29 is a block diagram illustrating an example of the configuration of a receiver (3DTV) forming the image transmitting/receiving system.
[Fig. 30] Fig. 30 illustrates an example of the structure of "AVC_video_descriptor" into which identification information indicating whether cropping information is 2D image or 3D image cropping information is inserted.
[Fig. 31] Fig. 31 illustrates an example of the principal data description contents of "AVC_video_descriptor".
[Fig. 32] Fig. 32 illustrates an example of the structure of "Stereo_Video_cropping_descriptor" into which identification information indicating whether cropping information is 2D image or 3D image cropping information is inserted.
[Fig. 33] Fig. 33 is a block diagram illustrating an example of the configuration of an image transmitting/receiving system in accordance with a fifth embodiment of this invention.
[Fig. 34] Fig. 34 is a block diagram illustrating an example of a transmission data generator in a broadcasting station forming the image transmitting/receiving system.
[Fig. 35] Fig. 35 illustrates processing (side-by-side mode) performed by a 2D signal processor, etc. of a receiver in the fifth embodiment.
[Fig. 36] Fig. 36 illustrates processing (top-and-bottom mode) performed by a 2D signal processor, etc. of a receiver in the fifth embodiment.
[Fig. 37] Fig. 37 is a block diagram illustrating an example of the configuration of a receiver (2DTV) forming the image transmitting/receiving system.
[Fig. 38] Fig. 38 illustrates, in three-dimensional image display utilizing binocular parallax, the relationship between the display positions of a left-eye image and a right-eye image of an object on a screen and the playback position of the three-dimensional image.
[Fig. 39] Fig. 39 illustrates examples of transmission formats (a side-by-side mode and a top-and-bottom mode) of three-dimensional image data.
[Fig. 40] Fig. 40 illustrates display image data generation processing in a reception side.
[Fig. 41] Fig. 41 illustrates side-by-side mode image processing performed by utilizing cropping information according to the related art.
[Fig. 42] Fig. 42 illustrates top-and-bottom mode image processing performed by utilizing cropping information according to the related art.

### Description of Embodiments

Modes for carrying out the invention (hereinafter referred to as "embodiments") will be described below. A description will be given in the following order.
1. First Embodiment
2. Second Embodiment
3. Third Embodiment
4. Fourth Embodiment
5. Fifth Embodiment

### <1. First Embodiment>

### "Image Transmitting/Receiving System"

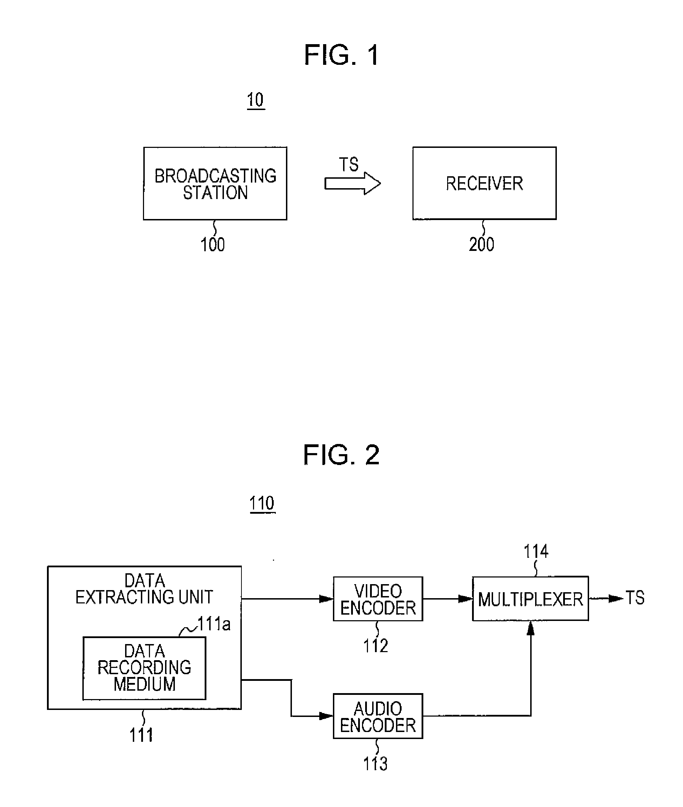

Fig. 1 illustrates an example of the configuration of an image transmitting/receiving system 10 according to a first embodiment. This image transmitting/receiving system 10 includes a broadcasting station 100 and a receiver 200. The broadcasting station 100 transmits through broadcasting waves a transport stream (multiplexed data stream) TS containing a video data stream including three-dimensional (3D) image data formed of left-eye image data and right-eye image data. The transmission format of this three-dimensional image data may be a side-by-side mode (see part (a) of Fig. 39) or a top-and-bottom mode (see part (b) of Fig. 39).

In this embodiment, it is assume that three-dimensional image data has a 1920 x 1080-pixel format. The broadcasting station 100 encodes the three-dimensional image data in units of 16x16 blocks. Accordingly, the broadcasting station 100 adds eight lines formed of blank data to the three-dimensional image data, making the image data be 1920-pixel x 1088-line image data, which is then encoded.

Three-dimensional (3D) display cropping information is inserted, together with two-dimensional (2D) display cropping information, into the header of the video data stream. Here, the 2D display cropping information forms first cropping information, while the 3D display cropping information forms second cropping information. In this embodiment, the video data stream is, for example, an H.264/AVC (Advanced Video Coding) stream.

In the transport stream TS, flag information indicating whether or not 3D display cropping information is inserted into the header of the video data stream is inserted into a higher layer of the video data stream. This flag information is inserted under a program map table, which serves as program specific information. More specifically, the flag information is inserted under a video elementary loop of a program map table or as a program descriptor of a program map table.

The receiver 200 receives a transport stream TS transmitted through broadcasting waves from the broadcasting station 100. The receiver 200 obtains side-by-side mode (see part (a) of Fig. 39) or top-and-bottom mode (see part (b) of Fig. 39) three-dimensional image data including left-eye image data and right-eye image data from the received transport stream TS.

As described above, in the broadcasting station 100, in order to perform encoding in units of 16x16 blocks, eight lines formed of blank data have been added, making the image data be 1920-pixel x 1088-line image data, which is then encoded. Accordingly, as three-dimensional image data after being decoded, the receiver 200 obtains 1920-pixel x 1088-line image data including eight lines formed of blank data.

If the receiver 200 is a television receiver 2DTV which does not support 3D display, i.e., a receiver which can perform only 2D display, it uses the 2D display cropping information, which is inserted into the header of the video data stream. That is, the receiver 200 crops, for example, the left-eye image data, from the received three-dimensional image on the basis of the 2D display cropping information so as to generate 2DTV display image data.

For example, if the side-by-side mode is employed, the receiver 200 crops, for example, 960-pixel x 1080-line left-eye image data from the 1920-pixel x 1080-line image data, which contains actual image data. Then, the receiver 200 performs scaling processing on this left-eye image data so as to generate 2DTV 1920-pixel x 1080-line display image data.

Also, if the top-and-bottom mode is employed, the receiver 200 crops, for example, 1920-pixel x 540-line left-eye image data from the 1920-pixel x 1080-line image data, which contains actual image data. Then, the receiver 200 performs scaling processing on this left-eye image data so as to generate 2DTV 1920-pixel x 1080-line display image data.

If the receiver 200 is a television receiver (3DTV) which can perform 3D display, it identifies, from flag information inserted into a higher layer of the video data stream of the transport stream TS, that 3D display cropping information has been inserted into the header of the video data stream. Then, the receiver 200 uses the 3D display cropping information inserted into a higher layer of the video data stream. That is, the receiver 200 crops 1920-pixel x 1080-line image data, which contains actual image data, from the received three-dimensional image data on the basis of the 3D display cropping information, thereby generating 3DTV left-eye and right-eye display image data.

For example, if the side-by-side mode is employed, the receiver 200 crops the 1920-pixel x 1080-line image data, which contains actual image data. Then, the receiver 200 divides this image data into a left frame and a right frame and performs scaling processing on the left and right frames, thereby generating 3DTV 1920-pixel x 1080-line left-eye and right-eye display image data.

Also, for example, if the top-and-bottom mode is employed, the receiver 200 crops the 1920-pixel x 1080-line image data, which contains actual image data. Then, the receiver 200 divides this image data into a top frame and a bottom frame and performs scaling processing on the top and bottom frames, thereby generating 3DTV 1920-pixel x 1080-line left-eye and right-eye display image data.

### "Example of Configuration of Transmission Data Generator"

Fig. 2 illustrates an example of the configuration of a transmission data generator 110 for generating the above-described transport stream TS in the broadcasting station 100. This transmission data generator 110 includes a data extracting unit (archive) 111, a video encoder 112, an audio encoder 113, and a multiplexer 114.

A data recording medium 111a, which is a disk recording medium, a semiconductor memory, etc., is, for example, detachably attached to the data extracting unit 111. In the data recording medium 111a, three-dimensional (3D) image data and corresponding sound data of a predetermined program, which is transmitted through the use of a transport stream TS, is recorded. The three-dimensional image data includes left-eye image data and right-eye image data. The transmission formats of the three-dimensional image data include, for example, a side-by-side mode (see part (a) of Fig. 39) and a top-and-bottom mode (see part (b) of Fig. 39). The data extracting unit 111 extracts and outputs three-dimensional image data and sound data from the data recording medium 111a.

The video encoder 112 performs encoding of H.264/AVC (Advanced Video Coding) on 3D image data output from the data extracting unit 111 so as to obtain encoded video data. The video encoder 112 also generates a video elementary stream (video data stream) including the encoded video data by using a stream formatter (not shown), which is provided subsequent to the video encoder 112.

In this case, the video encoder 112 inserts 2D display cropping information (first cropping information) and also inserts 3D display cropping information (second cropping information) into the header of the video data stream.

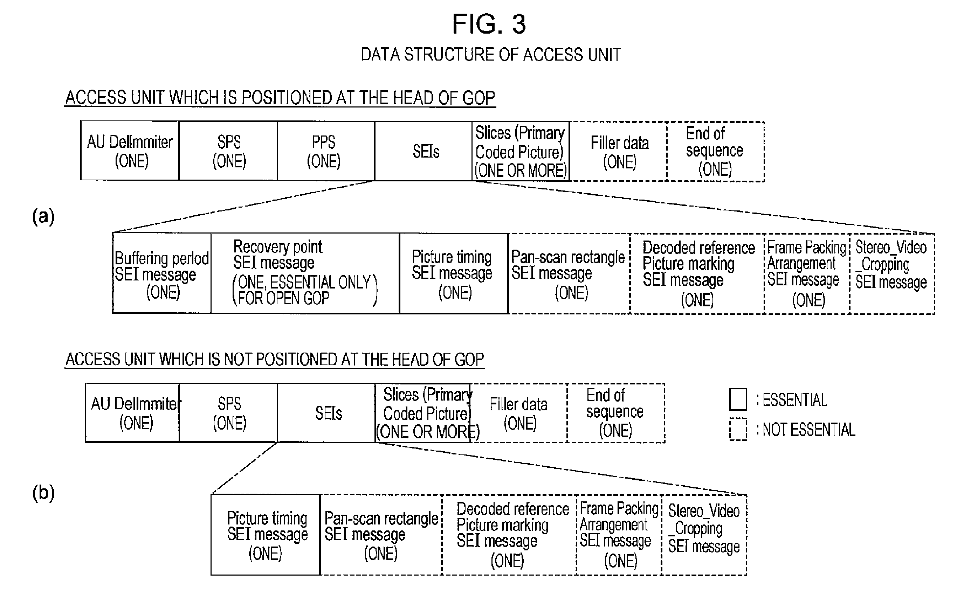

Part (a) and part (b) of Fig. 3 illustrate examples of the data structure of access units of the video data stream. In H.264, a picture is defined as a unit called an access unit. Part (a) of Fig. 3 illustrates the structure of an access unit which is positioned at the head of a GOP (Group Of Pictures). Part (b) of Fig. 3 illustrates the structure of an access unit which is not positioned at the head of a GOP.

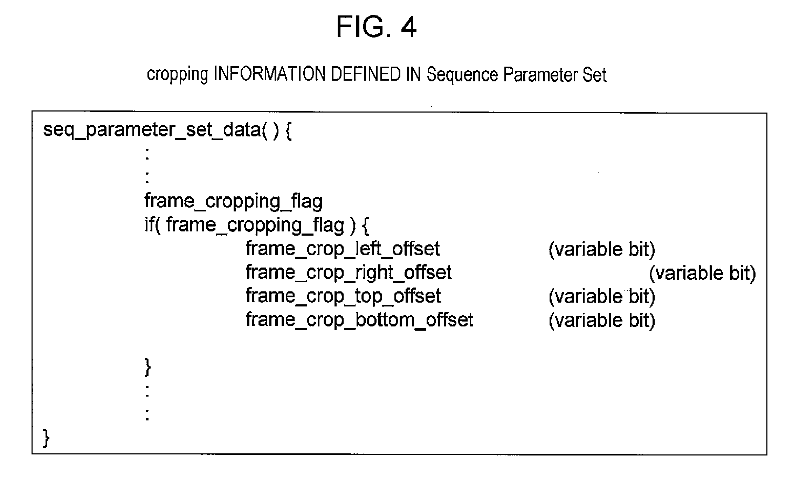

2D display cropping information is inserted into a SPS (Sequence Parameter Set) of the access unit. Fig. 4 illustrates the structure (Syntax) of cropping information defined in the SPS. In the SPS, flag information "frame_cropping_flag" indicates the presence or absence of cropping information. The cropping information is information which specifies a rectangular area, which serves as a cropping area to be cropped from image data.

In the SPS, "frame_crop_left_offset" indicates the horizontal start position, i.e., the left edge position; "frame_crop_right_offset" indicates the horizontal end position, i.e., the right edge position; "frame_crop_top_offset" indicates the vertical start position, i.e., the top edge position; and "frame_crop_bottom_offset" indicates the vertical end position, i.e., the bottom edge position. All the positions are represented by offset values from the top left position.

If image data is three-dimensional image data, "Frame Packing Arrangement Supplemental Enhancement Information (SEI) message" is inserted into SEIs of the access unit. This SEI includes type information indicating what type of transmission format is used for the three-dimensional image data.

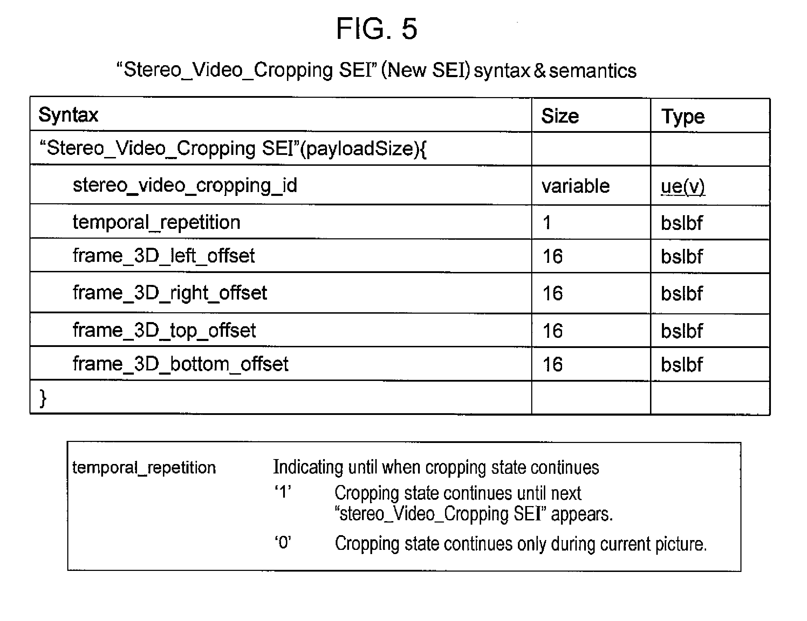

In this embodiment, "Stereo_Video_Cropping SEI" is newly defined in the SEIs of the access unit. Then, 3D display cropping information is inserted into this SEI. Fig. 5 illustrates an example of the structure (Syntax) and the principal data definition contents (semantics) of "Stereo_Video_Cropping SEI".

The "stereo_video_cropping_id" field is an identifier for identifying "Stereo_Video_Cropping SEI". A one-bit field "temporal_repetition" indicates until when the cropping state represented by the 3D display cropping information contained in this SEI continues. "1" indicates that the cropping state continues until a next "Stereo_Video_Cropping SEI" appears, and "0" indicates that the cropping state continues only during the current picture (access unit).

The provision of the information "temporal_repetition" enables the reception side to easily identify until when the cropping state represented by the 3D display cropping information contained in this SEI continues.

The 3D display cropping information contained in the "Stereo_Video_Cropping SEI", as well as the 2D display cropping information contained in the above-described SPS, is information which specifies a rectangular area, which serves as a cropping area to be cropped from image data. Thus, the configuration of the 3D display cropping information is similar to that of the 2D display cropping information.

The "frame_3D_left_offset" indicates the horizontal start position, i.e., the left edge position; "frame_3D_right_offset" indicates the horizontal end position, i.e., the right edge position; "frame_3D_top_offset" indicates the vertical start position, i.e., the top edge position; and "frame_3D_bottom_offset" indicates the vertical end position, i.e., the bottom edge position. All the positions are represented by offset values from the top left position.

Referring back to Fig. 2, the audio encoder 113 encodes sound data output from the data extracting unit 111 by using, for example, MPEG-2Audio AAC, so as to generate an audio elementary stream (audio data stream). The multiplexer 114 packetizes the elementary streams generated by the video encoder 112 and the audio encoder 113 and multiplexes the packetized streams so as to generate a transport stream (multiplexed data stream) TS.

Here, the multiplexer 114 inserts flag information into a higher layer of the video data stream. The flag information indicates whether 3D display cropping information is inserted into the header of the video data stream. In this embodiment, this flag information is inserted, for example, under a program map table, which serves as program specific information.

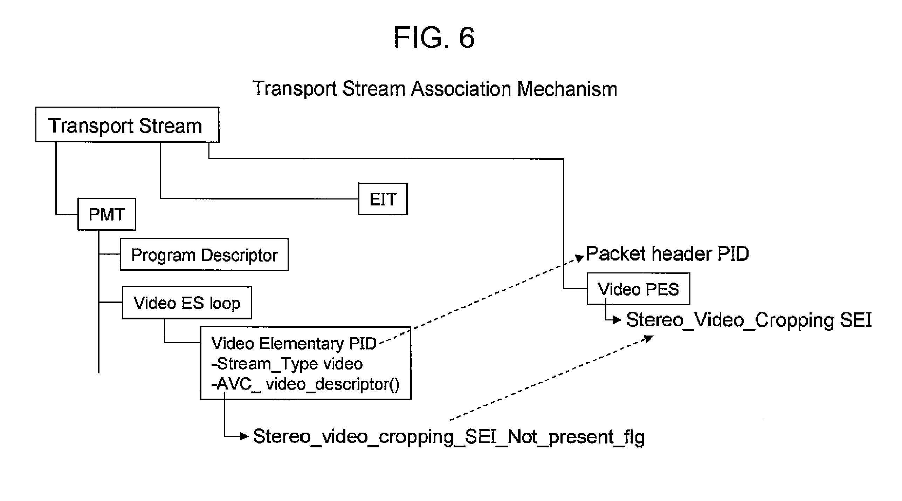

Fig. 6 illustrates an example of the configuration of a transport stream TS when flag information is inserted into a video elementary loop of the program map table. In this example of the configuration, a PES packet "Video PES" of a video elementary stream is included.

In the transport stream TS, PMT (ProgramMap Table) is contained as a PSI (Program Specific Information). This PSI is information indicating to which program each elementary stream contained in a transport stream TS belongs. The transport stream also includes an EIT (EventInformation Table), which serves as SI (Serviced Information), that manages event information in units of events.

The PMT includes a Program Descriptor which describes information concerning the entire program. The PMT also includes an elementary loop having information concerning each elementary stream. In this configuration, a video elementary loop (Video ES loop)) is contained.

In this elementary loop, information, such as a packet identifier (PID) and a stream type (Stream_Type), is provided for each elementary stream, and also, a descriptor which describes information related to that elementary stream is also disposed. In this example of the configuration, for simple representation for the drawing, information concerning audio streams is not shown in the drawing.

In this example of the configuration, "Stereo_Video_Cropping SEI" is newly defined in the header of the video data stream, and 3D display cropping information is inserted into this SEI, as described above. Then, in this example of the configuration, flag information indicating the presence of this SEI, i.e., the presence of 3D display cropping information, is inserted into "AVC_video_descriptor" contained in the video elementary loop (Video ES loop).

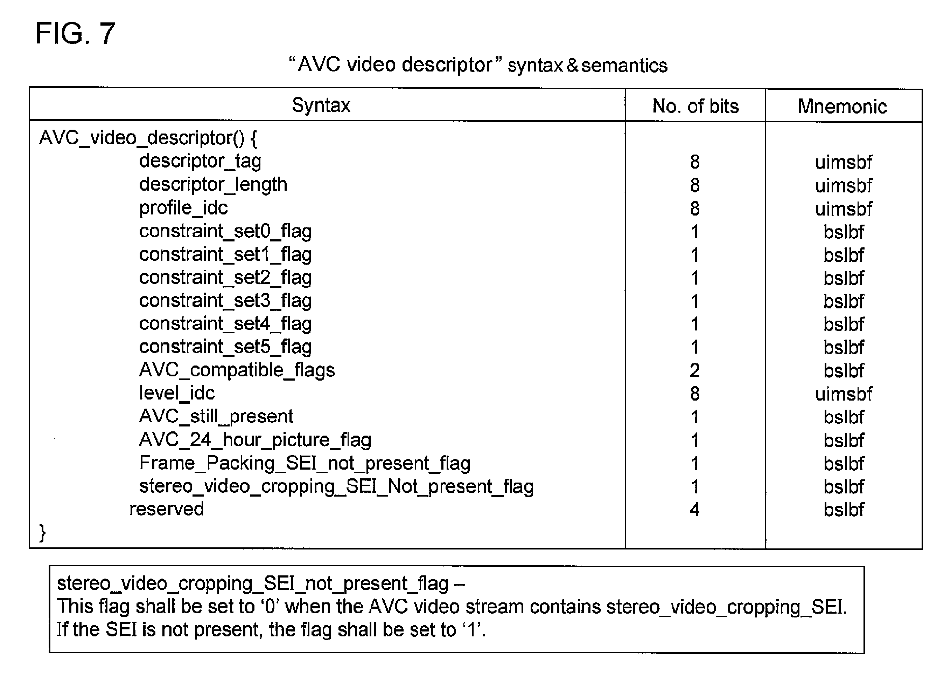

Fig. 7 illustrates an example of the structure (Syntax) and the principal data description contents (semantics) of the "AVC_video_descriptor". The descriptor itself is already contained in the H.264/AVC standards. In this configuration, in the descriptor, one-bit flag information "stereo_video_cropping_SEI_Not_present_flag" is newly defined.

This flag information indicates whether "Stereo_Video_Cropping SEI" is contained in the header of a video data stream, i.e., whether 3D display cropping information is inserted. "0" indicates that this SEI is included, and "1" indicates that this SEI is not included.

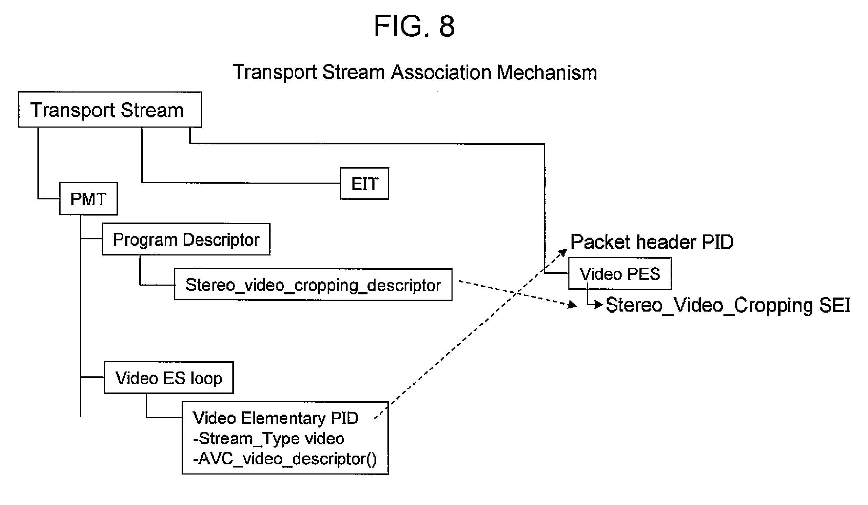

Fig. 8 illustrates an example of the configuration of a transport stream TS when flag information is inserted as a program descriptor of a program map table. In Fig. 8, a description will not be given of portions corresponding to those shown in Fig. 6.

In this example of the configuration, "Stereo_Video_Cropping SEI" is newly defined in the header of the video data stream, and 3D display cropping information is inserted into this SEI, as described above. Then, in this example of the configuration, a program descriptor "Stereo_Video_cropping_descriptor" containing flag information which indicates the presence of this SEI, i.e., the presence of 3D display cropping information, is newly defined.

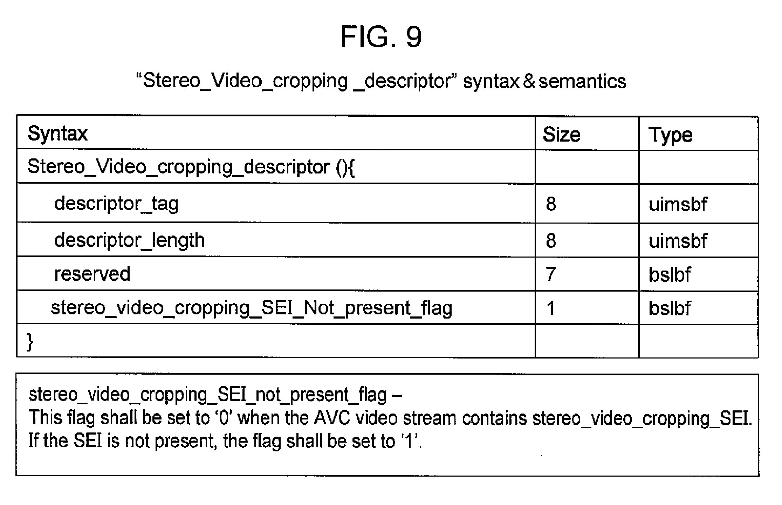

Fig. 9 illustrates an example of the structure (Syntax) and the principal data description contents (semantics) of "Stereo_Video_cropping_descriptor". The eight-bit "descriptor_tag" field indicates that this descriptor is "Stereo_Video_cropping_descriptor". The eight-bit "descriptor_length" field indicates the number of bytes of the fields after the "descriptor_length" field.

The "stereo_video_cropping_SEI_Not_present_flag" field is one-bit flag information, which is similar to the "stereo_video_cropping_SEI_Not_present_flag" which is newly defined in the above-described "AVC_video_descriptor". This flag information indicates whether "Stereo_Video_Cropping SEI" is contained in the header of a video data stream, i.e., whether 3D display cropping information is inserted. "0" indicates that this SEI is included, and "1" indicates that this SEI is not included.

The operation of the transmission data generator 110 shown in Fig. 2 will be briefly described below. Three-dimensional (3D) image data extracted from the data extracting unit 111 is supplied to the video encoder 112. The video encoder 112 encodes the image data by using H.264/AVC (Advanced Video Coding) so as to obtain encoded video data. The video encoder 112 also generates a video elementary stream (video data stream) including this encoded video data by using a stream formatter (not shown), which is provided subsequent to the video encoder 112.

In this case, the video encoder 112 inserts 2D display cropping information (first cropping information) and also inserts 3D display cropping information (second cropping information) into the header of the video data stream. The 2D display cropping information is inserted into the SPS of the access unit. In the SEIs of the access unit, "Stereo_Video_Cropping SEI" is newly defined, and the 3D display cropping information is inserted into this SEI.

When three-dimensional image data is output from the data extracting unit 111, sound data corresponding to the image data is also output from the data extracting unit 111. This sound data is supplied to the audio encoder 113. This audio encoder 113 encodes the sound data by using, for example, MPEG-2Audio AAC, so as to generate an audio elementary stream (audio data stream) including the encoded audio data.

The video elementary stream generated in the video encoder 112 is supplied to the multiplexer 114. The audio elementary stream generated in the audio encoder 113 is also supplied to the multiplexer 114. The multiplexer 114 packetizes the video elementary stream and the audio elementary stream supplied from the video encoder 112 and the audio encoder 113, respectively, and multiplexes the packetized streams so as to generate a transport stream (multiplexed data stream) TS.

In this case, the multiplexer 114 inserts flag information, which indicates whether 3D display cropping information is inserted into the header of the video data stream, into a higher layer of the video data stream. The flag information is inserted, for example, under a program map table, which serves as program specific information.

In the transmission data generator 110 shown in Fig. 2, as described above, the video encoder 112 inserts 3D display cropping information, together with 2D display cropping information, into the header of a video data stream. Accordingly, a 3DTV at a reception side is able to correctly perform image data cropping processing on the basis of the 3D display cropping information. Thus, the 3DTV at the reception side is able to correctly generate left-eye display image data and right-eye display image data, thereby enabling accurate three-dimensional display.

In the transmission data generator 110 shown in Fig. 2, the multiplexer 114 inserts, into a higher layer of the data stream, flag information indicating whether 3D display cropping information is contained in the header of the video data stream. Accordingly, the 3DTV at the reception side is able to identify the presence or absence of 3D display cropping information without analyzing the header of the video data stream.

### "Example of Configuration of Receiver"

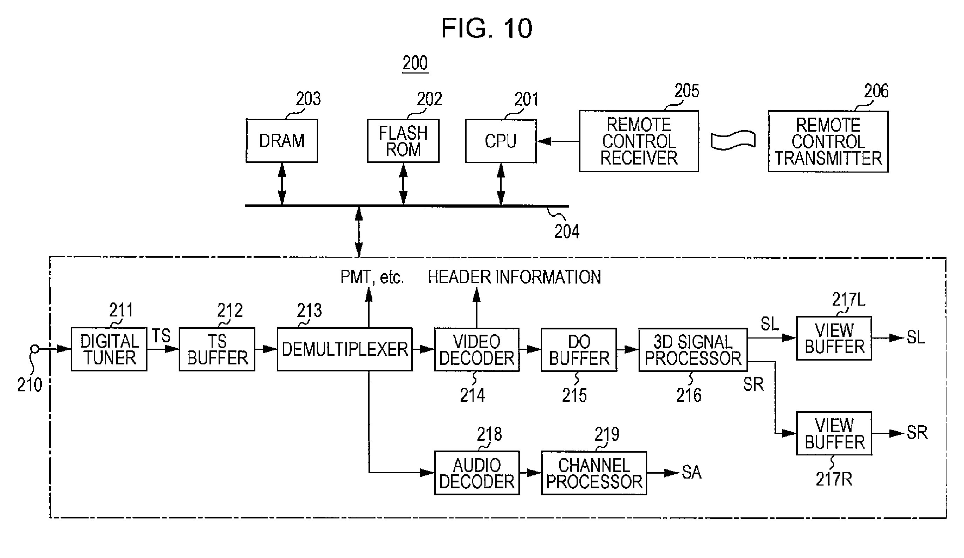

Fig. 10 illustrates an example of the configuration of the receiver 200. The receiver 200 is a television receiver (3DTV) which can perform 3D display. This receiver 200 includes a CPU 201, a flash ROM 202, a DRAM 203, an internal bus 204, a remote control receiver 205, and a remote control transmitter 206.

This receiver 200 also includes an antenna terminal 210, a digital tuner 211, a transport stream buffer (TS buffer) 212, and a demultiplexer 213. This receiver 200 also includes a video decoder 214, a display output buffer (DO buffer) 215, a 3D signal processor 216, view buffers 217L and 217R, an audio decoder 218, and a channel processor 219.

The CPU 201 controls the operations of all the components of the receiver 200. The flash ROM 202 stores control software therein and retains data. The DRAM 203 forms a work area for the CPU 201. The CPU 201 loads software or data read from the flash ROM 202 into the DRAM 203 and starts the software, thereby controlling the components of the receiver 200. The remote control receiver 205 receives a remote control signal (remote control code) transmitted from the remote control transmitter 206 and supplies the received remote control code to the CPU 201. The CPU 201 controls the components of the receiver 200 on the basis of the remote control code. The CPU 201, the flash ROM 202, and the DRAM 203 are connected to the internal bus 204.

The antenna terminal 210 receives a television broadcasting signal through a reception antenna (not shown). The digital tuner 211 processes the television broadcasting signal input into the antenna terminal 210 and outputs a predetermined transport stream (bit stream data) TS corresponding to a channel selected by a user. The transport stream buffer (TS buffer) 212 temporarily stores the transport stream TS output from the digital tuner 211.

The transport stream TS includes a video data stream containing left-eye image data and right-eye image data which employs the side-by-side or top-and-bottom mode. In the header of the video data stream, 3D display cropping information is inserted, together with 2D display cropping information. The 2D display cropping information is inserted into the SPS of an access unit. Additionally, the 3D display cropping information is inserted into "Stereo_Video_Cropping SEI", which is newly defined in the SEIs of the access unit.

In the transport stream TS, flag information "Stereo_video_cropping_SEI_Not_present_flag" is inserted into a higher layer of the video data stream. This flag information indicates whether 3D display cropping information is inserted into the header of the video data stream. This flag information is inserted and placed under a program map table, which serves as program specific information. More specifically, the flag information is inserted and placed under a video elementary loop of a program map table or as a program descriptor of a program map table.

The demultiplexer 213 extracts video and audio elementary streams from the transport stream TS, which is temporarily stored in the TS buffer 212. The demultiplexer 213 also extracts a program map table (PMT) from the transport stream TS and supplies information of this table to the CPU 201.

As described above, this table contains flag information ("Stereo_video_cropping_SEI_Not_present_flag") indicating whether 3D display cropping information is inserted into the header of the video data stream. The CPU 201 identifies, on the basis of this flag information, whether 3D display cropping information is contained in the header of the video data stream.

The video decoder 214 performs processing reverse to the processing performed by the video encoder 112 of the above-described transmission data generator 110. More specifically, the video decoder 214 decodes the encoded image data contained in the video elementary stream (video data stream) extracted by the demultiplexer 213 so as to obtain decoded three-dimensional image data.

As described above, in the transmission data generator 110 of the broadcasting station 100, in order to perform encoding in units of 16x16 blocks, eight lines formed of blank data have been added to the 1920 x 1080-pixel format, resulting in 1920-pixel x 1088-line image data, which have been encoded. Accordingly, as the three-dimensional image data after decoding, the video decoder 214 obtains 1920-pixel x 1088-line image data including eight-line blank data.

Additionally, the video decoder 214 extracts header information of the video data stream and supplies the extracted header information to the CPU 201. In this case, the 2D display cropping information is contained in the SPS of the access unit, and the 3D display cropping information is contained in "Stereo_Video_Cropping SEI", which is newly defined in the SEIs of the access unit.

The DO buffer 215 temporarily stores the three-dimensional image data obtained by the video decoder 214. Under the control of the CPU 201, the 3D signal processor 216 crops, on the basis of the 3D display cropping information, the 1920-pixel x 1080-line image data, which contains actual image data, from the three-dimensional image data stored in the DO buffer 215, thereby generating 3DTV left-eye display image data SL and right-eye display image data SR.

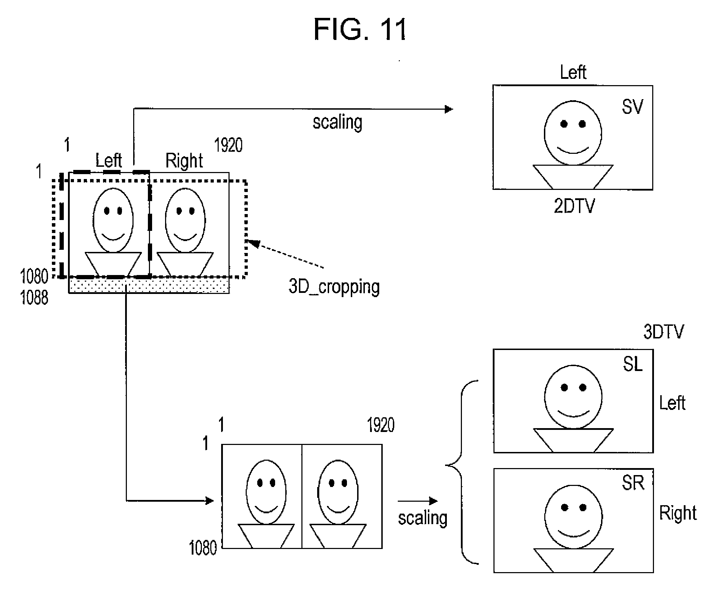

That is, if the side-by-side mode is employed, as shown in Fig. 11, the 3D signal processor 216 crops the 1920-pixel x 1088-line image data, which contains actual image data, from the 1920-pixel x 1080-line image data. The 3D signal processor 216 then divides this image data into left and right frames and performs horizontal scaling processing on each of the left and right frames, thereby generating 3DTV 1920-pixel x 1080-line left-eye display image data SL and right-eye display image data SR.

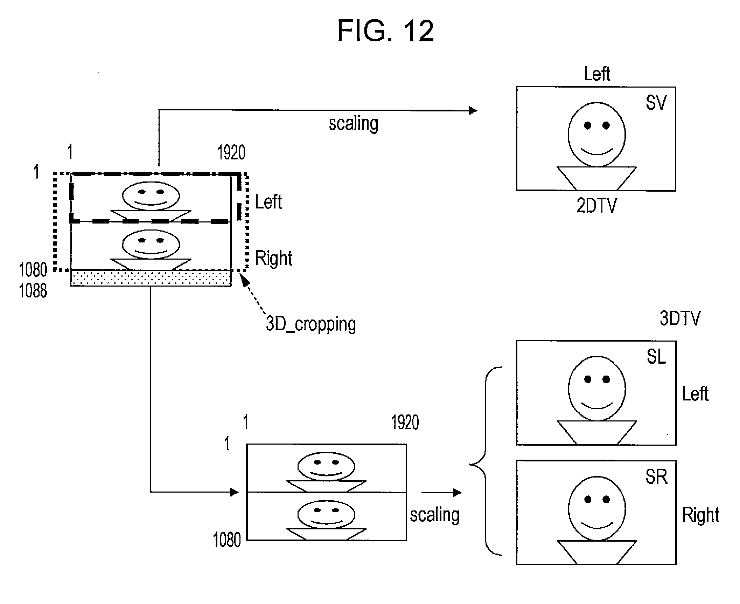

Alternatively, if the top-and-bottom mode is employed, as shown in Fig. 12, the 3D signal processor 216 crops the 1920-pixel x 1088-line image data, which contains actual image data, from the 1920-pixel x 1080-line image data. The 3D signal processor 216 then divides this image data into top and bottom frames and performs vertical scaling processing on each of the top and bottom frames, thereby generating 3DTV 1920-pixel x 1080-line left-eye display image data SL and right-eye display image data SR.

Referring back to Fig. 10, the view buffer 217L temporarily stores the 3DTV 1920-pixel x 1080-line left-eye display image data SL, and then outputs the image data to an image output unit, such as a display. Also, the view buffer 217R temporarily stores the 3DTV 1920-pixel x 1080-line right-eye display image data SR, and then outputs the image data to an image output unit, such as a display.

The audio decoder 218 performs processing reverse to the processing performed by the audio encoder 113 of the above-described transmission data generator 110. More specifically, the audio decoder 218 decodes the encoded sound data contained in the audio elementary stream extracted by the demultiplexer 213 so as to obtain decoded sound data. The channel processor 219 generates sound data SA of each channel for implementing, for example, 5.1 ch surround sound, from the sound data obtained in the audio decoder 218, and then outputs the sound data SA to a sound output unit, such as a speaker.

The operation of the receiver 200 will be briefly described below. A television broadcasting signal which has been input into the antenna terminal 210 is supplied to the digital tuner 211. This digital tuner 211 processes the television broadcasting signal and outputs a predetermined transport stream TS corresponding to a channel selected by a user. This transport stream TS is temporarily stored in the TS buffer 212.

The demultiplexer 213 extracts video and audio elementary streams from the transport stream TS, which is temporarily stored in the TS buffer 212. The demultiplexer 213 also extracts the program map table (PMT) from the transport stream TS. The information of this table is supplied to the CPU 201.

This table contains flag information "Stereo_video_cropping_SEI_Not_present_flag" indicating whether 3D display cropping information is contained in the header of the video data stream. The CPU 201 identifies, on the basis of the flag information, whether 3D display cropping information is contained in the header of the video data stream.

The video elementary stream (video data stream) extracted by the demultiplexer 213 is supplied to the video decoder 214. The video decoder 214 decodes the encoded image contained in the video elementary stream so as to obtain decoded three-dimensional image data. The three-dimensional image data is 1920-pixel x 1088-line image data including eight-line blank data. The three-dimensional image data is then temporarily stored in the DO buffer 215.

The video decoder 214 also extracts header information of the video data stream and supplies the header information to the CPU 201. In this case, 2D display cropping information is contained in the SPS, while 3D display cropping information is contained in the "Stereo_Video_Cropping SEI".

Under the control of the CPU 201, the 3D signal processor 216 crops, on the basis of the 3D display cropping information, the 1920-pixel x 1080-line image data, which contains actual image data, from the three-dimensional image data stored in the DO buffer 215. The 3D signal processor 216 then generates 3DTV 1920-pixel x 1088-line left-eye display image data SL and right-eye display image data SR. The 3DTV display image data SL and the 3DTV display image data SR are output to an image output unit, such as a display, through the view buffers 217L and 217R, respectively.

Additionally, the audio elementary stream extracted by the demultiplexer 213 is supplied to the audio decoder 218. This audio decoder 218 decodes the encoded sound data contained in the audio elementary stream so as to obtain decoded sound data. This sound data is supplied to the channel processor 219. The channel processor 219 generates sound data SA of each channel for implementing, for example, 5.1 ch surround sound, from the sound data supplied from the audio decoder 218. The sound data SA is then output to a sound output unit, such as a speaker.

As described above, in the receiver 200 shown in Fig. 10, the 3D signal processor 216 performs cropping on the basis of the 3D display cropping information inserted into the header of the video data stream. More specifically, the 3D signal processor 216 crops the 1920-pixel x 1080-line image data, which contains actual image data, from the three-dimensional image data. Accordingly, left-eye display image data and right-eye display image data are correctly generated, thereby enabling accurate 3D display.

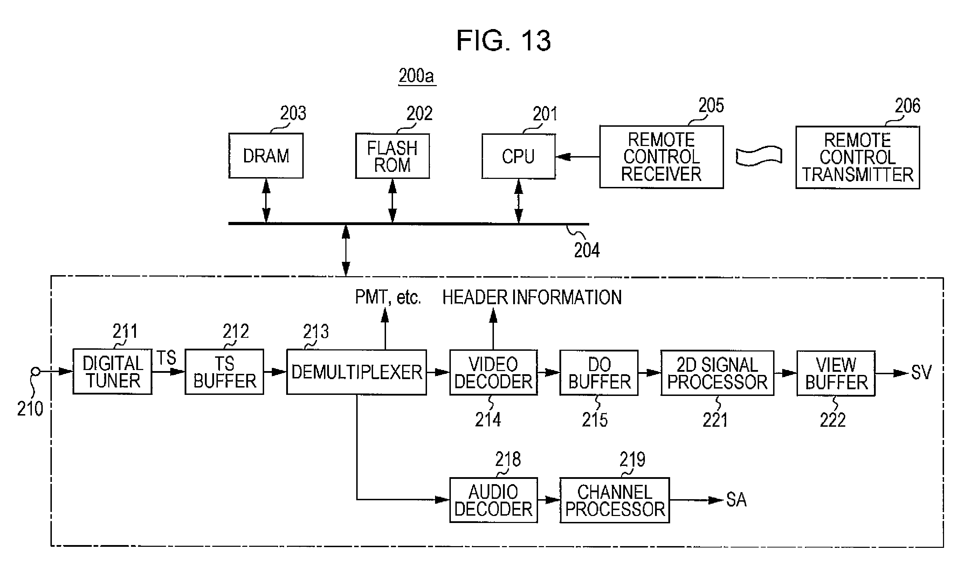

Note that Fig. 13 illustrates an example of the configuration of a receiver 200, which is television receiver (2DTV) that can perform only 2D display. Here, the receiver is designated by reference numeral 200a for the sake of convenience. In Fig. 13, elements corresponding to those shown in Fig. 10 are designated by like reference numerals, and an explanation thereof is omitted as appropriate.

In this receiver 200a, the demultiplexer 213 extracts video and audio elementary streams from the transport stream TS, which is temporarily stored in the TS buffer 212. The demultiplexer 213 also extracts the program map table (PMT) from the transport stream TS. The information of this table is supplied to the CPU 201.

This table contains flag information (Stereo_video_cropping_SEI_Not_present_flag) indicating whether 3D display cropping information is contained in the header of the video data stream. However, the CPU 201 of this receiver 200a ignores this flag information.

The video decoder 214 decodes the encoded image contained in the video elementary stream extracted by the demultiplexer 213 so as to obtain decoded three-dimensional image data. This three-dimensional image data is 1920-pixel x 1088-line image data including eight-line blank data. This three-dimensional image data is then temporarily stored in the DO buffer 215.

The video decoder 214 also extracts header information of the video data stream and supplies the header information to the CPU 201. In this case, 2D display cropping information is contained in the SPS of an access unit, while 3D display cropping information is contained in "Stereo_Video_Cropping SEI", which is newly defined in the SEIs of the access unit. However, the CPU 201 of this receiver 200a ignores the 3D display cropping information.

In the receiver 200a, the 3D signal processor 216 and the view buffers 217L and 217R of the above-described receiver 200 are substituted by a 2D signal processor 221 and a view buffer 222, respectively. Under the control of the CPU 201, the 2D signal processor 221 crops, for example, left-eye image data, on the basis of the 2D display cropping information, from the three-dimensional image data stored in the DO buffer 215 so as to generate 2DTV display image data SV.

For example, if the side-by-side mode is employed, as shown in Fig. 11, the 2D signal processor 221 crops, for example, 960-pixel x 1080-line left-eye image data, from 1920-pixel x 1080-line image data, which contains actual image data. Then, the 2D signal processor 221 performs scaling processing on the left-eye image data so as to generate 2DTV 1920-pixel x 1080-line display image data SV.

If, for example, the top-and-bottom mode is employed, as shown in Fig. 12, the 2D signal processor 221 crops, for example, 1920-pixel x 540-line left-eye image data, from 1920-pixel x 1080-line image data, which contains actual image data. Then, the 2D signal processor 221 performs scaling processing on the left-eye image data so as to generate 2DTV 1920-pixel x 1080-line display image data SV. The 2DTV display image data SV generated by the 2D signal processor 221 is output to an image output unit, such as a display, via the view buffer 222.

The other components of the receiver 200a are configured and operated as those of the receiver 200 shown in Fig. 10. In the receiver 200a, which is a television receiver (2DTV) that can perform only 2D display, the 2D signal processor 221 performs cropping on the basis of the 2D display cropping information inserted into the header of the video stream data so as to correctly generate display image data, thereby performing accurate 2D display.

### <2. Second Embodiment>

### "Image Transmitting/Receiving System"

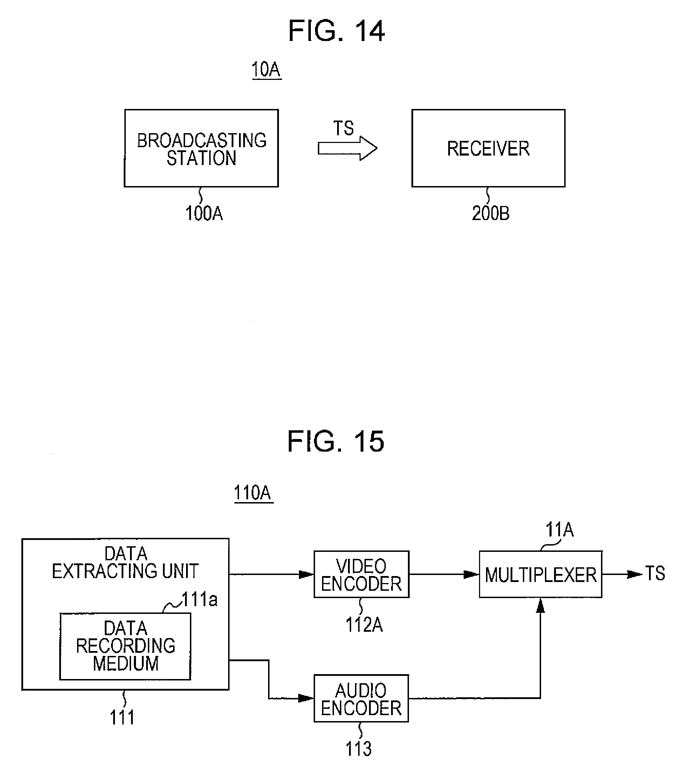

Fig. 14 illustrates an example of the configuration of an image transmitting/receiving system 10A in accordance with a second embodiment. The image transmitting/receiving system 10A includes a broadcasting station 100A and a receiver 200A. The broadcasting station 100A transmits through broadcasting waves a transport stream (multiplexed data stream data) TS including a video data stream containing three-dimensional (3D) image data having left-eye image data and right-eye image data. The transmission format of this 3D image data may be a side-by-side mode (see Fig. 39A) or a top-and-bottom mode (see Fig. 39B).

In this second embodiment, it is assumed that three-dimensional image data has a 1920 x 1080-pixel format. The broadcasting station 100A encodes this three-dimensional image data in units of 16x16 blocks. Accordingly, the broadcasting station 100A adds eight lines formed of blank data to the 3D image data, making the image data be 1920-pixel x 1088-line image data, which is then encoded.

Two-dimensional 2D display cropping information is inserted into the header of the video data stream. In this case, unlike the above-described first embodiment, three-dimensional (3D) display cropping information is not inserted into the header of the video data stream. Accordingly, in this second embodiment, unlike the above-described first embodiment, flag information indicating whether 3D display cropping information is contained in the header of the video data stream is not inserted into a higher layer of the video data stream.

The receiver 200A receives a transport stream TS transmitted through broadcasting waves from the broadcasting station 100A. The receiver 200A obtains side-by-side mode (see part (a) of Fig. 39) or top-and-bottom mode (see part (b) of Fig. 39) three-dimensional image data including left-eye image data and right-eye image data from the received transport stream TS.

As described above, in the broadcasting station 100A, in order to perform encoding in units of 16x16 blocks, eight lines formed of blank data are added to 1920-pixel x 1080-line image data, making the image data be 1920-pixel x 1088-line image data, which is then encoded. Accordingly, the receiver 200A obtains 1920-pixel x 1088-line image data including eight lines formed of blank data as the three-dimensional image data after decoding.

If the receiver 200A is a television receiver (2DTV) which does not support 3D display, i.e., a receiver which can perform only 2D display, it uses the 2D display cropping information inserted into the header of the video data stream. That is, the receiver 200A crops, for example, the left-eye image data, from the three-dimensional image data on the basis of the 2D display cropping information so as to generate 2DTV display image data.

For example, if the side-by-side mode is employed, the receiver 200A crops 960-pixel x 1080-line left-eye image data from the 1920-pixel x 1080-line image data, which contains actual image data. Then, the receiver 200A performs scaling processing on the 960-pixel x 1080-line left-eye image data so as to generate 2DTV 1920-pixel x 1080-line display image data.

Alternatively, if the top-and-bottom mode is employed, the receiver 200A crops 1920-pixel x 540-line left-eye image data from the 1920-pixel x 1080-line image data, which contains actual image data. Then, the receiver 200A performs scaling processing on the 1920-pixel x 540-line left-eye image data so as to generate 2DTV 1920-pixel x 1080-line display image data.

If the receiver 200A is a television receiver (3DTV) which can perform 3D display, it converts the 2D display cropping information inserted into the header of the video data stream into 3D display cropping information. Then, the receiver 200A crops 1920-pixel x 1080-line image data, which contains actual image data, from the three-dimensional image data on the basis of this 3D display cropping information, thereby generating 3DTV left-eye display image data and right-eye display image data.

For example, in the side-by-side mode, the 2D display cropping information is information which specifies a rectangular area for cropping, for example, 960-pixel x 1080-line left-eye image data, from the 1920-pixel x 1080-line image data. The receiver 200A converts the 2D display cropping information into information which specifies a rectangular area for cropping the entire 1920-pixel x 1080-line image data.

Then, the receiver 200A crops the 1920-pixel x 1080-line image data on the basis of the converted 3D display cropping information. The receiver 200A then divides this image data into a left frame and a right frame and performs scaling processing on each of the left and right frames, thereby generating 3DTV 1920-pixel x 1080-line left-eye display image data and right-eye display image data.

Additionally, for example, in the top-and-bottom mode, the 2D display cropping information is information which specifies a rectangular area for cropping, for example, 1920-pixel x 540-line left-eye image data, from the 1920-pixel x 1080-line image data. The receiver 200A then converts this 2D display cropping information into information which specifies a rectangular area for cropping the entire 1920-pixel x 1080-line image data.

Then, the receiver 200A crops the 1920-pixel x 1080-line image data on the basis of the converted 3D display cropping information. The receiver 200A then divides this image data into a top frame and a bottom frame and performs scaling processing on each of the top and bottom frames, thereby generating 3DTV 1920-pixel x 1080-line left-eye display image data and right-eye display image data.

### "Example of Configuration of Transmission Data Generator"

Fig. 15 illustrates an example of the configuration of a transmission data generator 110A for generating the above-described transport stream TS in the broadcasting station 100A. The transmission data generator 110A includes a data extracting unit (archive) 111, a video encoder 112A, an audio encoder 113, and a multiplexer 114A. In Fig. 15, elements corresponding to those shown in Fig. 2 are designated by like reference numerals, and a detailed explanation thereof is omitted as appropriate.

The video encoder 112A encodes three-dimensional image data output from the data extracting unit 111 by using H.264/AVC (Advanced Video Coding) so as to obtain encoded video data. The video encoder 112A also generates a video elementary stream (video data stream) including the encoded video data by using a stream formatter (not shown), which is provided subsequent to the video encoder 112A.

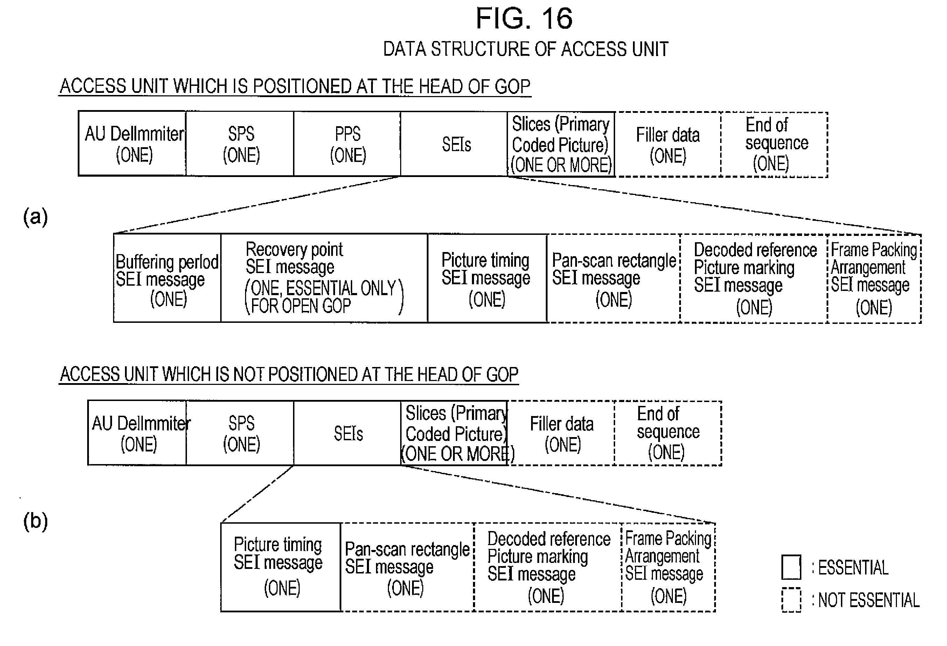

In this case, the video encoder 112A inserts 2D display cropping information (see Fig. 4) into the header of this video data stream. However, unlike the video encoder 112 of the transmission data generator 110 shown in Fig. 2, the video encoder 112A does not insert 3D display cropping information. Part (a) and part (b) of Fig. 16 illustrate examples of the data structure of access units of the video data stream. In H.264, a picture is defined as a unit called an access unit. Part (a) of Fig. 16 illustrates the structure of an access unit which is positioned at the head of a GOP (Group Of Pictures). Part (b) of Fig. 16 illustrates the structure of an access unit which is not positioned at the head of a GOP.

As in the data structures of the access units shown in part (a) and part (b) of Fig. 3, 2D display cropping information is inserted into a SPS (Sequence Parameter Set) of an access unit. Unlike the data structures of the access units shown in part (a) and part (b) of Fig. 3, "Stereo_Video_Cropping SEI" is not defined in the SEIs of the access unit.

The multiplexer 114A packetizes the elementary streams generated by the video encoder 112A and the audio encoder 113, and multiplexes the packetized streams so as to generate a transport stream (multiplexed data stream) TS. Unlike the multiplexer 114 of the transmission data generator 110 shown in Fig. 2, the multiplexer 114A does not insert, into a higher layer of the video data stream, flag information indicating whether 3D display cropping information is inserted into the header of the video data stream.

The other components of the transmission data generator 110A shown in Fig. 15 are configured and operated similarly to those of the transmission data generator 110 shown in Fig. 2. In the transmission data generator 110A shown in Fig. 15, the multiplexer 114A generates the following transport stream (multiplexed data stream) TS. That is, the transport stream TS includes a video data stream containing three-dimensional image data having left-eye image data and right-eye image data. Two-dimensional 2D display cropping information is inserted into the header of the video data stream.

### "Example of Configuration of Receiver"

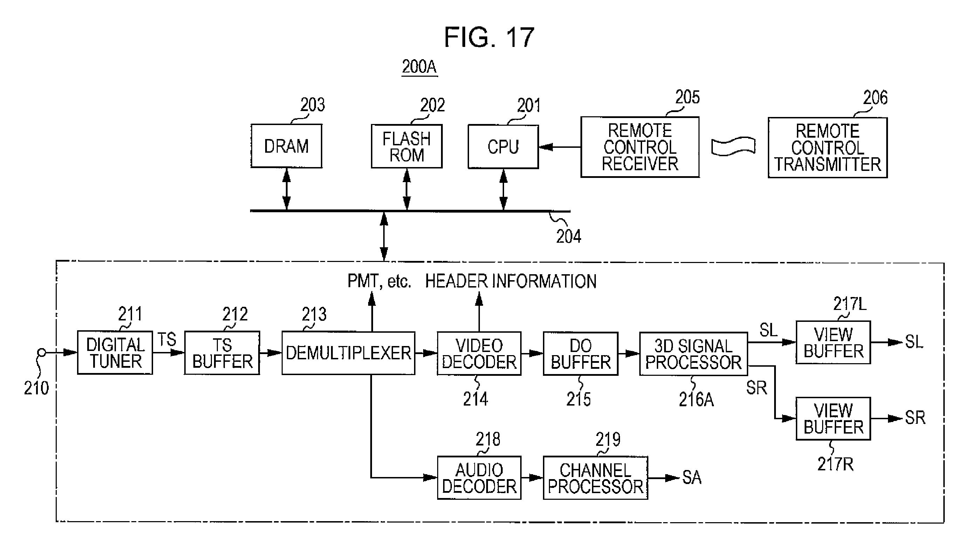

Fig. 17 illustrates an example of the configuration of the receiver 200A. This receiver 200A is a television receiver (3DTV) which can perform 3D display. In Fig. 17, elements corresponding to those shown in Fig. 10 are designated by like reference numerals, and an explanation thereof is omitted as appropriate.

The video decoder 214 decodes the encoded image data contained in the video elementary stream (video data stream) extracted by the demultiplexer 213 so as to obtain decoded three-dimensional image data. This three-dimensional image data is 1920-pixel x 1088-line image data including eight-line blank data. This three-dimensional image data is temporarily stored in the DO buffer 215.

The video decoder 214 also extracts header information of the video data stream and supplies the header information to the CPU 201. In this case, 2D display cropping information is contained in the SPS of an access unit.
Image data cropping processing performed by a 3D signal processor 216A, which will be discussed later, is controlled on the basis of the 2D display cropping information.

The CPU 201 converts the 2D display cropping information into 3D display cropping information. For example, if the transmission format of the three-dimensional image data is the side-by-side mode, the value "frame_crop_right_offset" indicating the horizontal end position, i.e., the right edge position, is doubled. Additionally, if the transmission format of the three-dimensional image data is the top-and-bottom mode, the value "frame_crop_bottom_offset" indicating the vertical end position, i.e., the bottom edge position, is doubled.

The 3D signal processor 216A crops, on the basis of the 3D display cropping information, 1920-pixel x 1080-line image data, which contains actual image data, from the three-dimensional image data stored in the DO buffer 215 so as to generate 3DTV left-eye display image data SL and right-eye display image data SR.

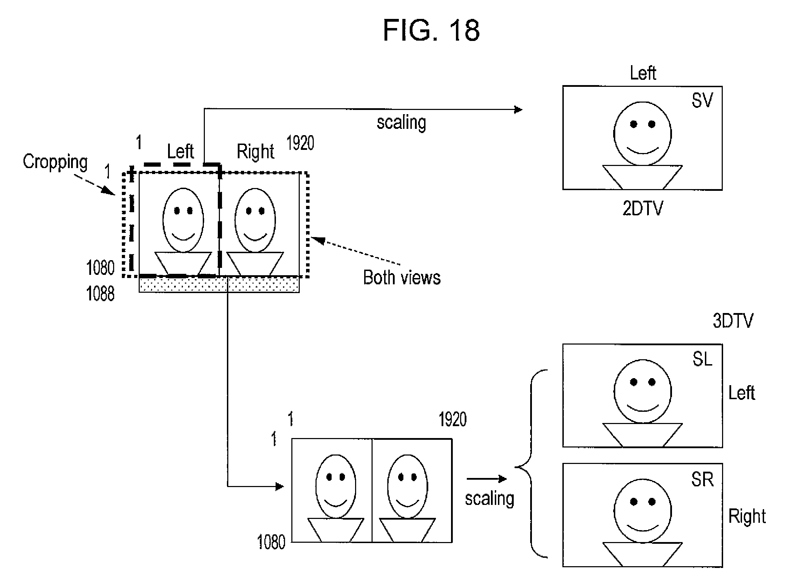

More specifically, if the side-by-side mode is employed, as shown in Fig. 18, the 3D signal processor 216A crops the 1920-pixel x 1088-line image data, which contains actual image data, from the 1920-pixel x 1080-line image data. The 3D signal processor 216A then divides this image data into left and right frames and performs horizontal scaling processing on each of the left and right frames, thereby generating 3DTV 1920-pixel x 1080-line left-eye display image data SL and right-eye display image data SR.

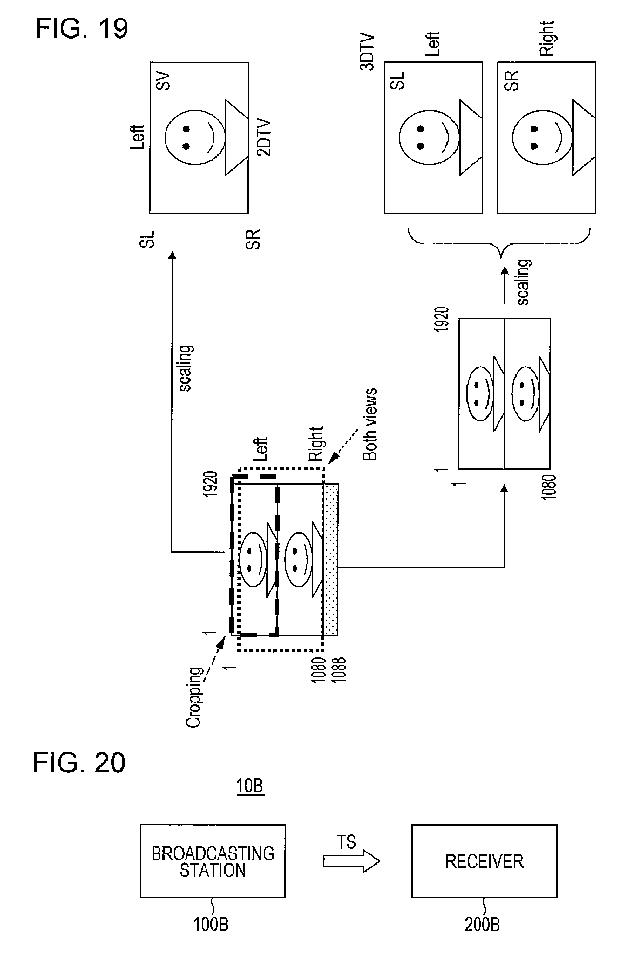

Alternatively, if the top-and-bottom mode is employed, as shown in Fig. 19, the 3D signal processor 216A crops the 1920-pixel x 1088-line image data, which contains actual image data, from the 1920-pixel x 1080-line image data. The 3D signal processor 216A then divides this image data into top and bottom frames and performs vertical scaling processing on each of the top and bottom frames, thereby generating 3DTV 1920-pixel x 1080-line left-eye display image data SL and right-eye display image data SR.

The other components of the receiver 200A shown in Fig. 17 are configured and operated similarly to those of the receiver 200 shown in Fig. 10. In the receiver 200A shown in Fig. 17, 2D display cropping information inserted into the header of the video data stream is converted into 3D display cropping information. Then, the 3D signal processor 216A performs cropping processing on the basis of the 3D display cropping information. More specifically, the 3D signal processor 216A crops 1920-pixel x 1080-line image data, which contains actual image data, from the three-dimensional image data. Thus, left-eye display image data and right-eye display image data are correctly generated, thereby enabling correct 3D display.

The processing performed by the 2D signal processor 221 of the receiver 200a shown in Fig. 13 is similar to that of the first embodiment. More specifically, the 2D signal processor 221 crops, for example, left-eye image data, on the basis of the 2D display cropping information, from the three-dimensional image data stored in the DO buffer 215 so as to generate 2DTV display image data SV.

For example, if the side-by-side mode is employed, as shown in Fig. 18, the 2D signal processor 221 crops, for example, 960-pixel x 1080-line left-eye image data, from 1920-pixel x 1080-line image data, which contains actual image data. Then, the 2D signal processor 221 performs scaling processing on the left-eye image data so as to generate 2DTV 1920-pixel x 1080-line display image data SV.

If, for example, the top-and-bottom mode is employed, as shown in Fig. 19, the 2D signal processor 221 crops, for example, 1920-pixel x 540-line left-eye image data, from 1920-pixel x 1080-line image data, which contains actual image data. Then, the 2D signal processor 221 performs scaling processing on the left-eye image data so as to generate 2DTV 1920-pixel x 1080-line display image data SV.

### <3. Third Embodiment>

### "Example of Image Transmitting/Receiving System"

Fig. 20 illustrates an example of the configuration of an image transmitting/receiving system 10B in accordance with a third embodiment. This image transmitting/receiving system 10B includes a broadcasting station 100B and a receiver 200B. The broadcasting station 100B transmits through broadcasting waves a transport stream (multiplexed data stream data) TS including a video data stream containing three-dimensional (3D) image data having left-eye image data and right-eye image data. The transmission format of this three-dimensional image data may be a side-by-side mode (see part (a) of Fig. 39) or a top-and-bottom mode (see part (b) of Fig. 39).

In this third embodiment, it is assumed that three-dimensional image data has a 1920 x 1080-pixel format. The broadcasting station 100B performs encoding on this three-dimensional image data in units of 16x16 blocks. Accordingly, the broadcasting station 100B adds eight lines formed of blank data to the 3D image data, making the image data be 1920-pixel x 1088-line image data, which is encoded.

Two-dimensional 2D display cropping information is inserted into the header of the video data stream. In this case, as in the second embodiment, three-dimensional (3D) display cropping information is not inserted into the header of the video data stream. Accordingly, as in the second embodiment, in the third embodiment, flag information indicating whether 3D display cropping information is contained in the header of the video data stream is not inserted into a higher layer of the video data stream.

The receiver 200B receives a transport stream TS transmitted through broadcasting waves from the broadcasting station 100B. The receiver 200B obtains side-by-side mode (see part (a) of Fig. 39) or top-and-bottom mode (see part (b) of Fig. 39) three-dimensional image data including left-eye image data and right-eye image data from the received transport stream TS.

As described above, in the broadcasting station 100B, in order to perform encoding in units of 16x16 blocks, eight lines formed of blank data are added to 1920-pixel x 1080-line image data, making the image data be 1920-pixel x 1088-line image data, which is then encoded. Accordingly, the receiver 200B obtains 1920-pixel x 1088-line image data including eight lines formed of blank data as the three-dimensional image data after decoding.

If the receiver 200B is a television receiver (2DTV) which does not support 3D display, i.e., a television receiver which can perform only 2D display, it uses the 2D display cropping information inserted into the header of the video data stream. That is, the receiver 200B crops, for example, the left-eye image data, from the received three-dimensional image on the basis of the 2D display cropping information so as to generate 2DTV display image data.

For example, if the side-by-side mode is employed, the receiver 200B crops 960-pixel x 1080-line left-eye image data from the 1920-pixel x 1080-line image data, which contains actual image data. Then, the receiver 200B performs scaling processing on this left-eye image data so as to generate 2DTV 1920-pixel x 1080-line image data.

Alternatively, if the top-and-bottom mode is employed, the receiver 200B crops 1920-pixel x 540-line left-eye image data from the 1920-pixel x 1080-line image data, which contains actual image data. Then, the receiver 200B performs scaling processing on this left-eye image data so as to generate 2DTV 1920-pixel x 1080-line image data.

On the other hand, if the receiver 200B is a television receiver (3DTV) which can perform 3D display, it performs image data cropping processing on the basis of the 2D display cropping information so as to generate one of left-eye display image data and right-eye display image data, e.g., left-eye display image data. The receiver 200B then generates the other one of the left-eye display image data and the right-eye display image data, e.g., the right-eye display image data, on the basis of the image data which remains after performing cropping processing based on the 2D display cropping information.

For example, in the side-by-side mode, the 2D display cropping information is information which specifies a rectangular area for cropping, for example, 960-pixel x 1080-line left-eye image data, from the 1920-pixel x 1080-line image data. The receiver 200B crops, on the basis of the 2D display cropping information, 960-pixel x 1080-line left-eye image data from the 1920-pixel x 1080-line image data, which contains actual image data. Then, the receiver 200B performs horizontal scaling on this left-eye image data so as to generate 3DTV 1920-pixel x 1080-line left-eye display image data.

Then, the receiver 200B crops the remaining 960-pixel x 1080-line image data, e.g., right-eye image data, from the 1920-pixel x 1080-line image data. Then, the receiver 200B performs horizontal scaling on this right-eye image data so as to generate 3DTV 1920-pixel x 1080-line right-eye display image data.

On the other hand, for example, in the top-and-bottom mode, the 2D display cropping information is information which specifies a rectangular area for cropping, for example, 1920-pixel x 540-line left-eye image data, from the 1920-pixel x 1080-line image data. The receiver 200B crops, on the basis of this information, 1920-pixel x 540-line left-eye image data from the 1920-pixel x 1080-line image data, which contains actual image data. Then, the receiver 200B performs vertical scaling on this left-eye image data so as to generate 3DTV 1920-pixel x 1080-line left-eye display image data.

Then, the receiver 200B crops the remaining 1920-pixel x 540-line image data, e.g., right-eye image data, from the 1920-pixel x 1080-line image data. The receiver 200B then performs vertical scaling on this right-eye image data so as to generate 3DTV 1920-pixel x 1080-line right-eye display image data.

### "Example of Configuration of Transmission Data Generator"

A transmission data generator for generating the above-described transport stream TS in the broadcasting station 100B is similarly configured as the transmission data generator 110A of the above-described second embodiment, though a detailed description thereof is not given.

### "Example of Configuration of Receiver"

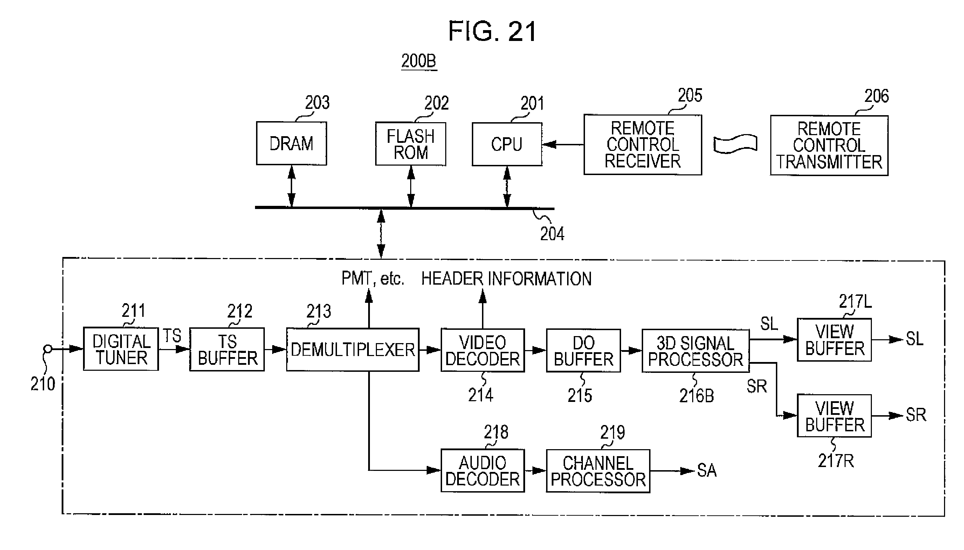

Fig. 21 illustrates an example of the configuration of the receiver 200B. The receiver 200B is a television receiver (3DTV) which can perform 3D display. In Fig. 21, elements corresponding to those shown in Fig. 10 are designated by like reference numerals, and a detailed explanation thereof is omitted as appropriate.

The video decoder 214 decodes the encoded image data contained in the video elementary stream (video data stream) extracted by the demultiplexer 213 so as to obtain decoded three-dimensional image data. This three-dimensional image data is 1920-pixel x 1088-line image data including eight-line blank data. This three-dimensional image data is temporarily stored in the DO buffer 215.

The video decoder 214 also extracts header information of the video data stream and supplies the header information to the CPU 201. In this case, 2D display cropping information is contained in the SPS of an access unit. Image data cropping processing performed by a 3D signal processor 216B, which will be discussed later, is controlled on the basis of the 2D display cropping information.

The CPU 201 generates, on the basis of the 2D display cropping information, remaining-area cropping information that specifies a rectangular area for cropping the remaining image data. For example, if the transmission format of the three-dimensional image data is the side-by-side mode, the value "frame_crop_right_offset" indicating the horizontal end position, i.e., the right edge position, is doubled so as to change the value into "alternative_view_horizontal_edge", thereby generating remaining-area cropping information.

This remaining-area cropping information includes "frame_crop_top_offset", "frame_crop_bottom_offset", "frame_crop_right_offset + 1", and "alternative_view_horizontal_edge". The value "frame_crop_right_offset + 1" indicates the horizontal start position, i.e., the left edge position; "alternative_view_horizontal_edge" indicates the horizontal end position, i.e., the right edge position; "frame_crop_top_offset" indicates the vertical start position, i.e., the top edge position; and "frame_crop_bottom_offset" indicates the vertical end position, i.e., the bottom edge position. All the positions are represented by offset values from the top left position.

Additionally, if the transmission format of the three-dimensional image data is the top-and-bottom mode, the value "frame_crop_bottom_offset" indicating the vertical end position, i.e., the bottom edge position, is doubled so as to change the value into "alternative_view_vertical_edge", thereby generating remaining-area cropping information.

The remaining-area cropping information includes "frame_crop_bottom_offset + 1", "alternative_view_vertical_edge", "frame_crop_left_offset", and "frame_crop_right_offset". The "frame_crop_left_offset" indicates the horizontal start position, i.e., the left edge position; "frame_crop_right_offset" indicates the horizontal end position, i.e., the right edge position; "frame_crop_bottom_offset + 1" indicates the vertical start position, i.e., the top edge position; and "alternative_view_vertical_edge" indicates the vertical end position, i.e., the bottom edge position. All the positions are represented by offset values from the top left position.

The 3D signal processor 216B performs image data cropping processing on the basis of the 2D display cropping information so as to generate one of left-eye display image data and right-eye display image data, e.g., left-eye display image data. Further, the 3D signal processor 216B performs image data cropping processing on the basis of the remaining-area cropping information so as to generate the other one of the left-eye display image data and the right-eye display image data, e.g., the right-eye display image data.

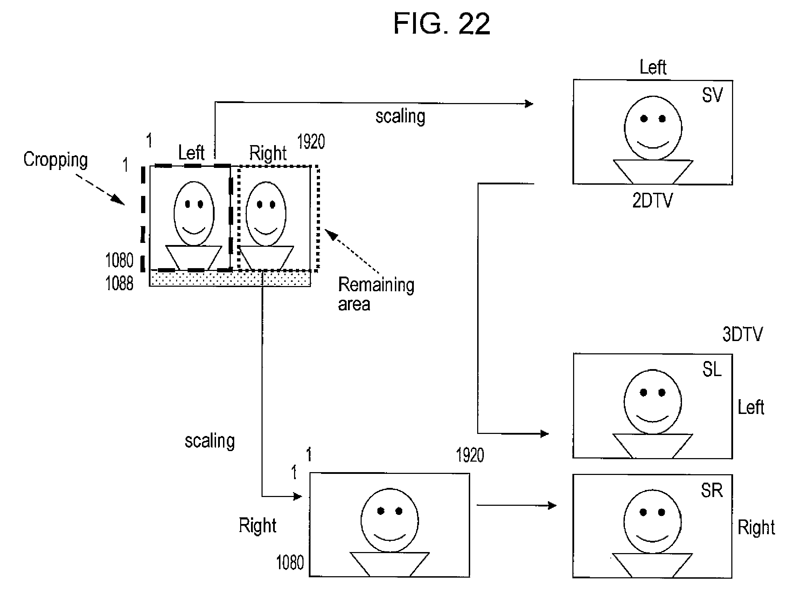

For example, in the side-by-side mode, the 2D display cropping information is information which specifies a rectangular area for cropping, for example, 960-pixel x 1080-line left-eye image data, from the 1920-pixel x 1080-line image data. The 3D signal processor 216B crops, as shown in Fig. 22, on the basis of the 2D display cropping information, 960-pixel x 1080-line left-eye image data from the 1920-pixel x 1080-line image data, which contains actual image data. Then, the 3D signal processor 216B performs horizontal scaling processing on this left-eye image data so as to generate 3DTV 1920-pixel x 1080-line left-eye display image data.

Additionally, the 3D signal processor 216B crops, as shown in Fig. 22, the remaining 960-pixel x 1080-line image data, e.g., right-eye image data, from the 1920-pixel x 1080-line image data on the basis of the remaining-area cropping information. Then, the 3D signal processor 216B performs horizontal scaling processing on this right-eye image data so as to generate 3DTV 1920-pixel x 1080-line right-eye display image data.

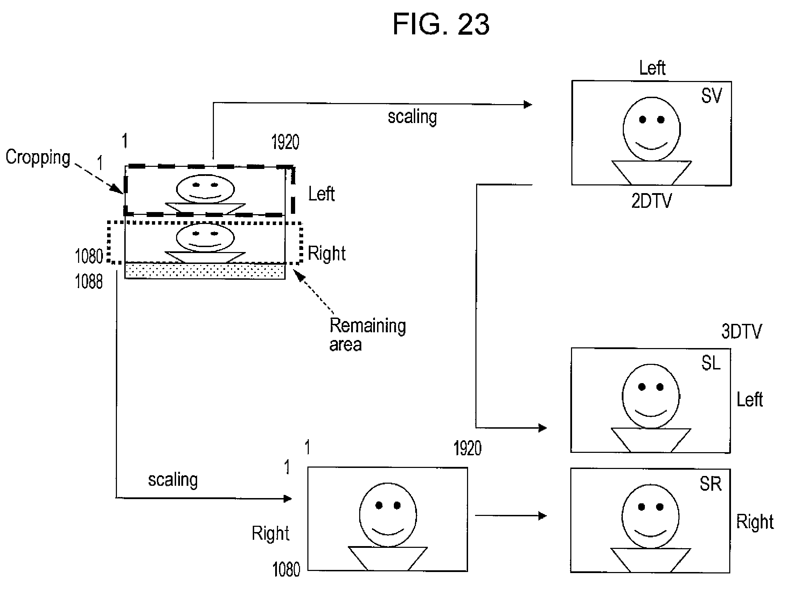

Additionally, for example, in the top-and-bottom mode, the 2D display cropping information is information which specifies a rectangular area for cropping, for example, 1920-pixel x 540-line left-eye image data, from the 1920-pixel x 1080-line image data. The 3D signal processor 216B crops, as shown in Fig. 23, on the basis of the 2D display cropping information, 1920-pixel x 540-line left-eye image data from the 1920-pixel x 1080-line image data, which contains actual image data. Then, the 3D signal processor 216B performs vertical scaling on this left-eye image data so as to generate 3DTV 1920-pixel x 1080-line left-eye display image data.

Then, the 3D signal processor 216B crops, as shown in Fig. 23, the remaining 1920-pixel x 540-line image data, e.g., right-eye image data, from the 1920-pixel x 1080-line image data on the basis of the remaining-area cropping information. The 3D signal processor 216B then performs vertical scaling on this right-eye image data so as to generate 3DTV 1920-pixel x 1080-line right-eye display image data.

The other components of the receiver 200B shown in Fig. 21 are configured and operated similarly to those of the receiver 200 shown in Fig. 10. In the receiver 200B shown in Fig. 21, image data is cropped on the basis of the 2D display cropping information contained in the video data stream so as to generate one of left-eye display image data and right-eye display image data. Also in the receiver 200B, the other one of the left-eye display image data and the right-eye display image data is generated on the basis of the image data which remains after performing cropping processing based on the 2D display cropping information. Thus, left-eye display image data and right-eye display image data are correctly generated, thereby enabling correct 3D display.

The processing performed by the 2D signal processor 221 of the receiver 200a shown in Fig. 13 is similar to that of the second embodiment. More specifically, the 2D signal processor 221 crops, for example, left-eye image data, on the basis of the 2D display cropping information, from the three-dimensional image data stored in the DO buffer 215 so as to generate 2DTV display image data SV.

For example, if the side-by-side mode is employed, as shown in Fig. 22, the 2D signal processor 221 crops, for example, 960-pixel x 1080-line left-eye image data, from 1920-pixel x 1080-line image data, which contains actual image data. Then, the 2D signal processor 221 performs scaling processing on this left-eye image data so as to generate 2DTV 1920-pixel x 1080-line display image data SV.

Alternatively, if, for example, the top-and-bottom mode is employed, as shown in Fig. 23, the 2D signal processor 221 crops, for example, 1920-pixel x 540-line left-eye image data, from 1920-pixel x 1080-line image data, which contains actual image data. Then, the 2D signal processor 221 performs scaling processing on this left-eye image data so as to generate 2DTV 1920-pixel x 1080-line display image data SV.

### <4. Fourth Embodiment>

### "Image Transmitting/Receiving System"

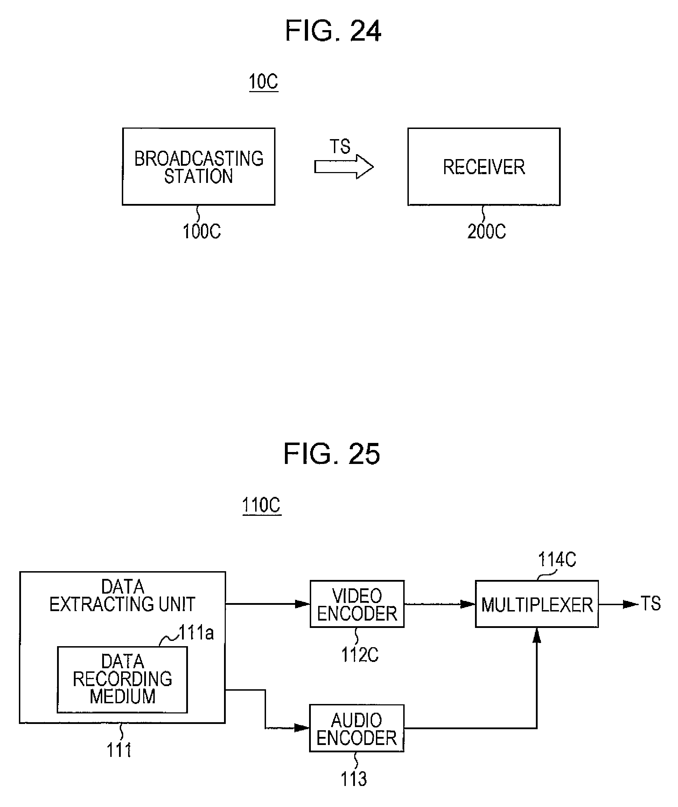

Fig. 24 illustrates an example of the configuration of an image transmitting/receiving system 10C in accordance with a fourth embodiment. This image transmitting/receiving system 10C includes a broadcasting station 100C and a receiver 200C. The broadcasting station 100C transmits through broadcasting waves a transport stream (multiplexed data stream data) TS including a video data stream containing three-dimensional (3D) image data having left-eye image data and right-eye image data. The transmission format of this three-dimensional image data may be a side-by-side mode (see part (a) of Fig. 39) or a top-and-bottom mode (see part (b) of Fig. 39).

In this embodiment, it is assumed that three-dimensional image data has a 1920 x 1080-pixel format. The broadcasting station 100C performs encoding on this three-dimensional image data in units of 16x16 blocks. Accordingly, the broadcasting station 100C adds eight lines formed of blank data to the three-dimensional image data, making the image data be 1920-pixel x 1088-line image data, which is then encoded.

2D image cropping information or 3D image cropping information is inserted into the header of the video data stream. The 2D image cropping information is information which specifies a rectangular area for cropping, for example, 1920-pixel x 1080-line image data, which contains actual image data. The 3D image cropping information is information which specifies a rectangular area for cropping, for example, a left-eye image data area or a right-eye image data area, from 1920-pixel x 1080-line image data. Identification information indicating whether the cropping information is 2D image cropping information or 3D image cropping information is inserted into the header of the video data stream. In this embodiment, the video data stream is, for example, an H.264/AVC (Advanced Video Coding) stream.

The receiver 200C receives a transport stream TS transmitted through broadcasting waves from the broadcasting station 100C. The receiver 200C obtains side-by-side mode (see part (a) of Fig. 39) or top-and-bottom mode (see part (b) of Fig. 39) three-dimensional image data including left-eye image data and right-eye image data from the received transport stream TS.

As described above, in the broadcasting station 100C, in order to perform encoding in units of 16x16 blocks, eight lines formed of blank data are added to 1920-pixel x 1080-line image data, making the image data be 1920-pixel x 1088-line image data, which is then encoded. Accordingly, the receiver 200C obtains 1920-pixel x 1088-line image data including eight lines formed of blank data as the three-dimensional image data after decoding.

If the receiver 200C is a television receiver (3DTV) which can perform 3D display, it identifies on the basis of the identification information inserted into the header of the video data stream whether the cropping information is 2D or 3D cropping information. The receiver 200C then crops data from the received three-dimensional image data on the basis of the cropping information so as to generate 3DTV left-eye display image data and right-eye display image data.

For example, if the cropping information is 2D cropping information and if the side-by-side mode is employed, the receiver 200C crops 1920-pixel x 1080-line image data, which contains actual image data, on the basis of this 2D image cropping information. Then, the receiver 200C divides the image data into a left frame and a right frame, and performs horizontal scaling processing on each of the left and right frames so as to generate 3DTV 1920-pixel x 1080-line left-eye display image data and right-eye display image data.

Additionally, if the cropping information is 2D cropping information and if the top-and-bottom mode is employed, the receiver 200C crops 1920-pixel x 1080-line image data, which contains actual image data, on the basis of this 2D image cropping information. Then, the receiver 200C divides the image data into a top frame and a bottom frame, and performs vertical scaling processing on each of the left and right frames so as to generate 3DTV 1920-pixel x 1080-line left-eye display image data and right-eye display image data.

If the cropping information is 3D cropping information, the receiver 200C performs the following processing (1), which is similar to the processing of the second embodiment, or the following processing (2), which is similar to the processing of the third embodiment, so as to generate 3DTV 1920-pixel x 1080-line left-eye display image data and 3DTV 1920-pixel x 1080-line right-eye display image data.

### "Processing (1)"

For example, if the receiver 200C is a television receiver (3DTV) which can perform 3D display, it converts the 3D image cropping information inserted into the header of the video data stream into 2D image cropping information. Then, the receiver 200C crops 1920-pixel x 1080-line image data from the three-dimensional image data on the basis of this 2D image cropping information, thereby generating 3DTV left-eye display image data and right-eye display image data.

For example, in the side-by-side mode, the 3D image cropping information is information which specifies a rectangular area for cropping, for example, 960-pixel x 1080-line left-eye image data, from the 1920-pixel x 1080-line image data. The receiver 200C then converts this 3D image cropping information into 2D image cropping information which specifies a rectangular area for cropping the entire 1920-pixel x 1080-line image data.

Then, the receiver 200C crops the 1920-pixel x 1080-line image data on the basis of the converted 2D image cropping information. The receiver 200C then divides this image data into a left frame and a right frame and performs scaling processing on each of the left and right frames, thereby generating 3DTV 1920-pixel x 1080-line left-eye display image data and right-eye display image data.

Alternatively, for example, in the top-and-bottom mode, the 3D image cropping information is information which specifies a rectangular area for cropping, for example, 1920-pixel x 540-line left-eye image data, from the 1920-pixel x 1080-line image data. The receiver 200C then converts this 3D image cropping information into 2D image cropping information which specifies a rectangular area for cropping the entire 1920-pixel x 1080-line image data.

Then, the receiver 200C crops the 1920-pixel x 1080-line image data, which is the actual image data, on the basis of the converted 2D image cropping information. The receiver 200C then divides this image data into a top frame and a bottom frame and performs scaling processing on each of the top and bottom frames, thereby generating 3DTV 1920-pixel x 1080-line left-eye display image data and right-eye display image data.

### "Processing (2)"

If the receiver 200C is a television receiver (3DTV) which can perform 3D display, it performs image data cropping processing on the basis of the 3D image cropping information so as to generate one of left-eye display image data and right-eye display image data, e.g., left-eye display image data. The receiver 200C then generates the other one of the left-eye display image data and the right-eye display image data, e.g., the right-eye display image data, on the basis of the image data which remains after performing cropping processing based on the 3D image cropping information.

For example, in the side-by-side mode, the 3D image cropping information is information which specifies a rectangular area for cropping, for example, 960-pixel x 1080-line left-eye image data, from the 1920-pixel x 1080-line image data. The receiver 200C crops, on the basis of the 3D image cropping information, 960-pixel x 1080-line left-eye image data from the 1920-pixel x 1080-line image data. Then, the receiver 200C performs horizontal scaling on this left-eye image data so as to generate 3DTV 1920-pixel x 1080-line left-eye display image data.

Then, the receiver 200C crops the remaining 960-pixel x 1080-line image data, e.g., right-eye image data, from the 1920-pixel x 1080-line image data, which contains actual image data. Then, the receiver 200C performs horizontal scaling on this right-eye image data so as to generate 3DTV 1920-pixel x 1080-line right-eye display image data.

Alternatively, for example, in the top-and-bottom mode, the 3D image cropping information is information which specifies a rectangular area for cropping, for example, 1920-pixel x 540-line left-eye image data, from the 1920-pixel x 1080-line image data. The receiver 200C crops, on the basis of the 3D image cropping information, 1920-pixel x 540-line left-eye image data from the 1920-pixel x 1080-line image data, which contains actual image data. Then, the receiver 200C performs vertical scaling on this left-eye image data so as to generate 3DTV 1920-pixel x 1080-line left-eye display image data.

Then, the receiver 200C crops the remaining 1920-pixel x 540-line image data, e.g., right-eye image data, from the 1920-pixel x 1080-line image data. The receiver 200C then performs vertical scaling on this right-eye image data so as to generate 3DTV 1920-pixel x 1080-line right-eye display image data.

### "Example of Configuration of Transmission Data Generator"

Fig. 25 illustrates an example of the configuration of a transmission data generator 110C for generating the above-described transport stream TS in the broadcasting station 100C. The transmission data generator 110C includes a data extracting unit (archive) 111, a video encoder 112C, an audio encoder 113, and a multiplexer 114C. Elements corresponding to those shown in Fig. 2 are designated by like reference numerals, and a detailed description is omitted as appropriate.

The video encoder 112C encodes three-dimensional image data output from the data extracting unit 111 by using H.264/AVC (Advanced Video Coding) so as to obtain encoded video data. The video encoder 112C also generates a video elementary stream (video data stream) including the encoded video data by using a stream formatter (not shown), which is provided subsequent to the video encoder 112C.

In this case, the video encoder 112C inserts the above-described 2D image cropping information or 3D image cropping information into the header of this video data stream (see Fig. 4). The video encoder 112C also inserts identification information indicating whether the cropping information is 2D image cropping information or 3D image cropping information.

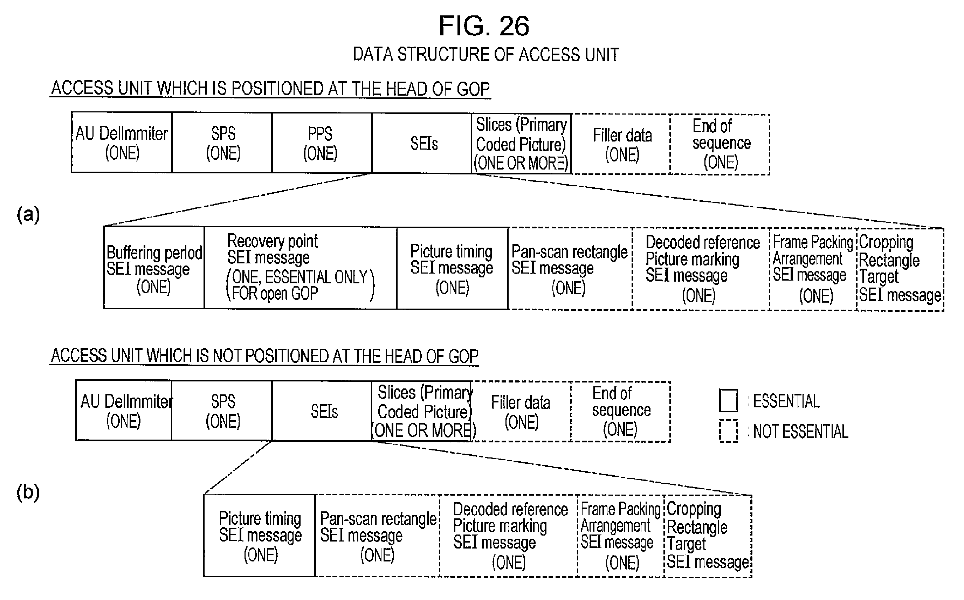

Part (a) and part (b) of Fig. 26 illustrate examples of the data structure of access units of the video data stream. In H.264, a picture is defined as a unit called an access unit. Part (a) of Fig. 26 illustrates the structure of an access unit which is positioned at the head of a GOP (Group Of Pictures). Part (b) of Fig. 26 illustrates the structure of an access unit which is not positioned at the head of a GOP. The cropping information is inserted into the SPS of an access unit.

If image data is three-dimensional image data, "Frame Packing Arrangement SEI message" is inserted into SEIs of the access unit. The SEI includes type information indicating what type of transmission format is used for the three-dimensional image data.

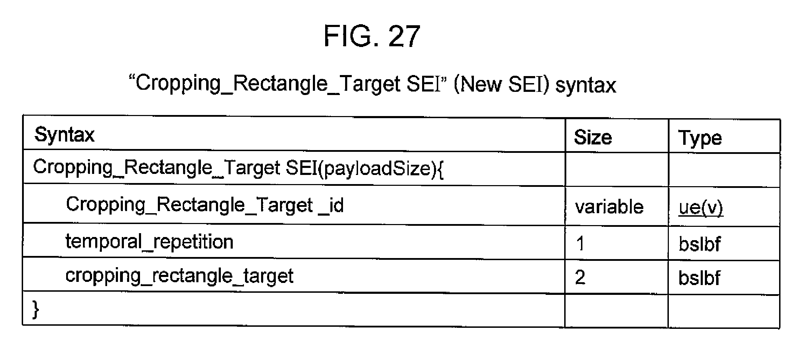

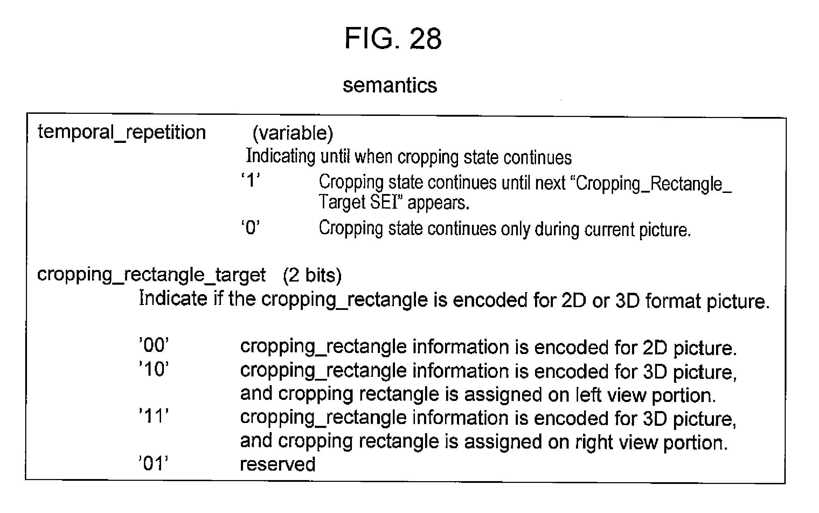

Also, in this embodiment, "Cropping_Rectangle_Target SEI" is newly defined in the SEIs of an access unit. In this SEI, identification information indicating whether the cropping information is 2D or 3D image cropping information is inserted. Fig. 27 and 28 respectively illustrate an example of the structure (Syntax) and an example of the principal data definition contents (semantics) of "Cropping_Rectangle_Target SEI".

The "Cropping_Rectangle_Target_id" field is an identifier for identifying the "Cropping_Rectangle_Target SEI". A one-bit field "temporal_repetition" indicates until when the cropping state represented by the cropping information continues. "1" indicates that the cropping state continues until a next "Cropping_Rectangle_Target SEI" appears, and "0" indicates that the cropping state continues only during the current picture (access unit).

The two-bit "cropping_rectangle_target" field is identification information indicating whether cropping information is 2D or 3D image cropping information. "00" indicates that cropping information is 2D image cropping information. "10" indicates that cropping information is 3D image cropping information and that the specified rectangular area corresponds to a left-eye area. "11" indicates that cropping information is 3D image cropping information and that the specified rectangular area corresponds to a right-eye area.

The multiplexer 114C packetizes elementary streams generated by the video encoder 112C and the audio encoder 113, and multiplexes the streams so as to generate a transport stream (multiplexed data stream) TS. Unlike the multiplexer 114 of the transmission data generator 110 shown in Fig. 2, the multiplexer 114C does not insert, into a higher layer of the video data stream, flag information indicating whether 3D display cropping information is contained in the header of the video data stream.

The other components of the transmission data generator 110C shown in Fig. 25 are configured and operated similarly to those of the transmission data generator 110 shown in Fig. 2. In the transmission data generator 110C shown in Fig. 25, the multiplexer 114C generates the following transport stream (multiplexed data stream) TS. That is, the transport stream TS includes a video data stream containing three-dimensional image data having left-eye image data and right-eye image data. 2D or 3D image cropping information and identification information thereof are inserted into the header of the video data stream.

As described above, in the transmission data generator 110C shown in Fig. 25, the video encoder 112C inserts identification information indicating whether cropping information is 2D or 3D image cropping information into the header of a video data stream. This enables a 3DTV at the reception side to easily identify that cropping information is 2D or 3D image cropping information and to perform suitable processing by using this cropping information.

### "Example of Configuration of Receiver"

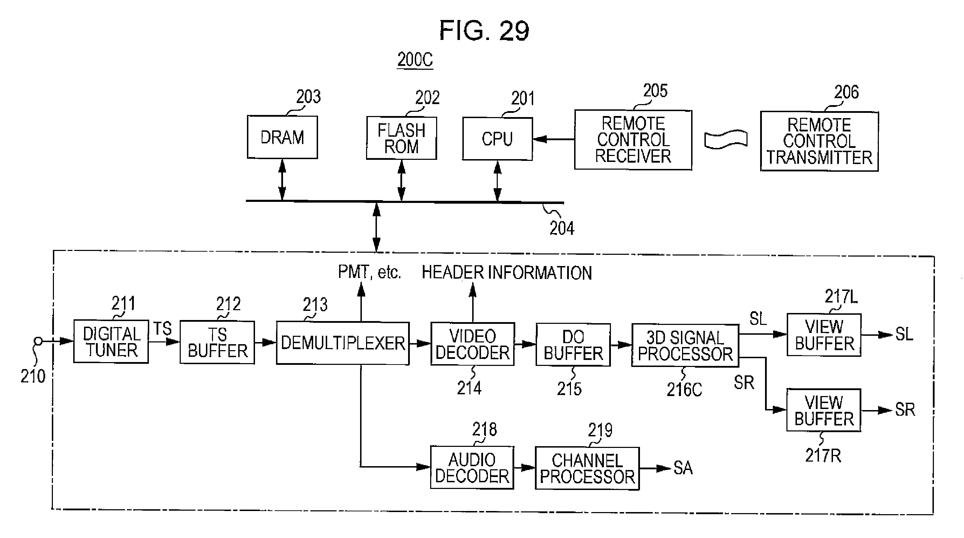

Fig. 29 illustrates an example of the configuration of the receiver 200C. This receiver 200C is a television receiver (3DTV) which can perform 3D display. In Fig. 29, elements corresponding to those shown in Fig. 10 are designated by like reference numerals, and an explanation thereof is omitted as appropriate.

The video decoder 214 performs decoding processing on the encoded image data contained in the video elementary stream (video data stream) extracted by the demultiplexer 213 so as to obtain decoded three-dimensional image data. This three-dimensional image data is 1920-pixel x 1088-line image data including eight-line blank data. This three-dimensional image data is temporarily stored in the DO buffer 215.

The video decoder 214 also extracts header information of the video data stream and supplies the header information to the CPU 201. In this case, 2D or 3D image cropping information is contained in the SPS of an access unit. Also, identification information indicating whether cropping information is 2D or 3D image cropping information is inserted into "Cropping_Rectangle_Target SEI", which is newly defined in the SEIs of an access unit. Image data cropping processing performed by a 3D signal processor 216C, which will be discussed later, is controlled on the basis of the cropping information and the identification information.

If the cropping information is 2D image cropping information, the 3D signal processor 216C crops, on the basis of the 2D image cropping information, 1920-pixel x 1080-line image data, which contains actual image data, from the three-dimensional image data stored in the DO buffer 215 so as to generate 3DTV left-eye display image data SL and right-eye display image data SR.

More specifically, if the side-by-side mode is employed, as shown in Fig. 11, the 3D signal processor 216C crops the 1920-pixel x 1088-line image data, which contains actual image data, from the 1920-pixel x 1080-line image data. The 3D signal processor 216C then divides this image data into left and right frames and performs horizontal scaling processing on each of the left and right frames, thereby generating 3DTV 1920-pixel x 1080-line left-eye display image data SL and right-eye display image data SR.

Alternatively, if the top-and-bottom mode is employed, as shown in Fig. 12, the 3D signal processor 216C crops the 1920-pixel x 1088-line image data, which contains actual image data, from the 1920-pixel x 1080-line image data. The 3D signal processor 216C then divides this image data into top and bottom frames and performs vertical scaling processing on each of the top and bottom frames, thereby generating 3DTV 1920-pixel x 1080-line left-eye display image data SL and right-eye display image data SR.

On the other hand, if the cropping information is 3D image cropping information, the 3D signal processor 216C performs, for example, the following processing (1) or the following processing (2).

### "Processing (1)"

The CPU 201 converts the 3D image cropping information into 2D image cropping information. For example, if the transmission format of the three-dimensional image data is the side-by-side mode, the value "frame_crop_right_offset" indicating the horizontal end position, i.e., the right edge position, is doubled. Also, for example, if the transmission format of the three-dimensional image data is the top-and-bottom mode, the value "frame_crop_bottom_offset" indicating the vertical end position, i.e., the bottom edge position, is doubled.

The 3D signal processor 216C crops, on the basis of the converted 2D image cropping information, 1920-pixel x 1080-line image data, which contains actual image data, from the three-dimensional image data stored in the DO buffer 215 so as to generate 3DTV left-eye display image data SL and right-eye display image data SR.

More specifically, if the side-by-side mode is employed, as shown in Fig. 18, the 3D signal processor 216C crops the 1920-pixel x 1088-line image data, which contains actual image data, from the 1920-pixel x 1080-line image data. The 3D signal processor 216C then divides this image data into left and right frames and performs horizontal scaling processing on each of the left and right frames, thereby generating 3DTV 1920-pixel x 1080-line left-eye display image data SL and right-eye display image data SR.

Alternatively, if the top-and-bottom mode is employed, as shown in Fig. 19, the 3D signal processor 216C crops the 1920-pixel x 1088-line image data, which contains actual image data, from the 1920-pixel x 1080-line image data. The 3D signal processor 216C then divides this image data into top and bottom frames and performs vertical scaling processing on each of the top and bottom frames, thereby generating 3DTV 1920-pixel x 1080-line left-eye display image data SL and right-eye display image data SR.

### "Processing (2)"

The CPU 201 generates, on the basis of 3D image cropping information, remaining-area cropping information that specifies a rectangular area for cropping remaining image data. The 3D signal processor 216C performs image data cropping processing on the basis of the 3D image cropping information so as to generate one of left-eye display image data and right-eye display image data, e.g., left-eye display image data. The 3D signal processor 216C also performs image data cropping processing on the basis of the remaining-area cropping information so as to generate the other one of the left-eye display image data and the right-eye display image data, e.g., the right-eye display image data.

In the side-by-side mode, for example, the 3D image cropping information is information which specifies a rectangular area for cropping, for example, 960-pixel x 1080-line left-eye image data, from the 1920-pixel x 1080-line image data.

The 3D signal processor 216C crops, as shown in Fig. 22, on the basis of this 3D image cropping information, 960-pixel x 1080-line left-eye image data from the 1920-pixel x 1080-line image data, which contains actual image data. Then, the 3D signal processor 216C performs horizontal scaling on this left-eye image data so as to generate 3DTV 1920-pixel x 1080-line left-eye display image data.

Then, the 3D signal processor 216C crops, as shown in Fig. 22, the remaining 960-pixel x 1080-line image data, e.g., right-eye image data, from the 1920-pixel x 1080-line image data on the basis of the remaining-area cropping information. Then, the 3D signal processor 216C performs horizontal scaling on this right-eye image data so as to generate 3DTV 1920-pixel x 1080-line right-eye display image data.

Alternatively, in the top-and-bottom mode, for example, the 3D display cropping information is information which specifies a rectangular area for cropping, for example, 1920-pixel x 540-line left-eye image data, from the 1920-pixel x 1080-line image data, which contains actual image data.

The 3D signal processor 216C crops, as shown in Fig. 23, on the basis of the 3D display cropping information, 1920-pixel x 540-line left-eye image data from the 1920-pixel x 1080-line image data, which contains actual image data. Then, the 3D signal processor 216C performs vertical scaling on this left-eye image data so as to generate 3DTV 1920-pixel x 1080-line left-eye display image data.

Then, the 3D signal processor 216C crops, as shown in Fig. 23, the remaining 1920-pixel x 540-line image data, e.g., right-eye image data, from the 1920-pixel x 1080-line image data on the basis of the remaining-area cropping information. The 3D signal processor 216C then performs vertical scaling on this right-eye image data so as to generate 3DTV 1920-pixel x 1080-line right-eye display image data.

The other components of the receiver 200C shown in Fig. 29 are configured and operated similarly to those of the receiver 200 shown in Fig. 10. In the receiver 200C shown in Fig. 29, image data is suitably cropped on the basis of cropping information and identification information contained in a video data stream, the identification information indicating whether the cropping information is 2D or 3D image cropping information. Thus, left-eye display image data and right-eye display image data are correctly generated, thereby enabling correct 3D display.

Note that, in the fourth embodiment, identification information indicating whether cropping information, which is inserted into the header of a video data stream, is 2D or 3D image cropping information is also inserted into the header of the video data stream. That is, in the fourth embodiment, "Cropping_Rectangle_Target SEI" is newly defined, and in this SEI, identification information is inserted.

However, identification information may be inserted into a higher layer of the video data stream, for example, under a program map table.

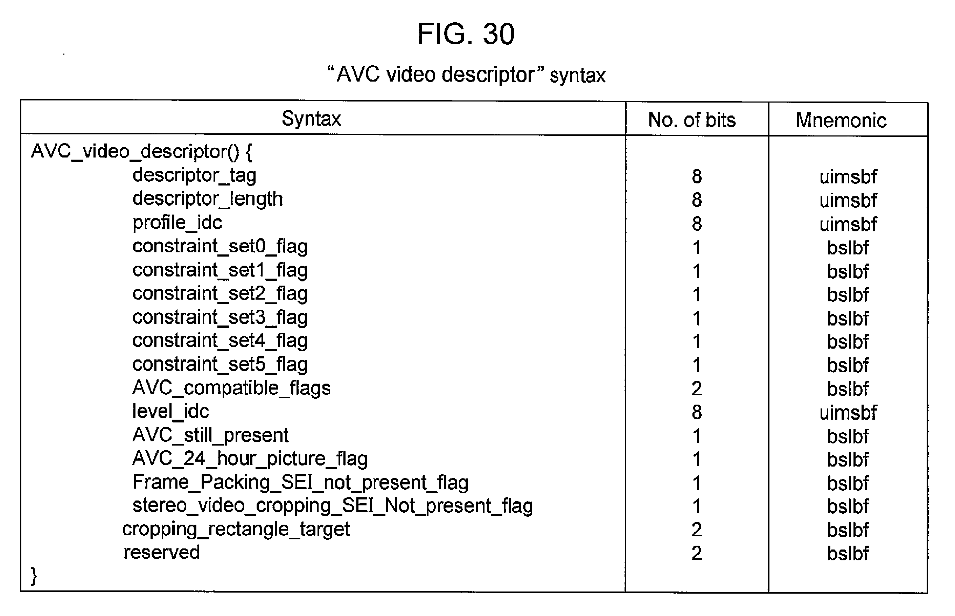

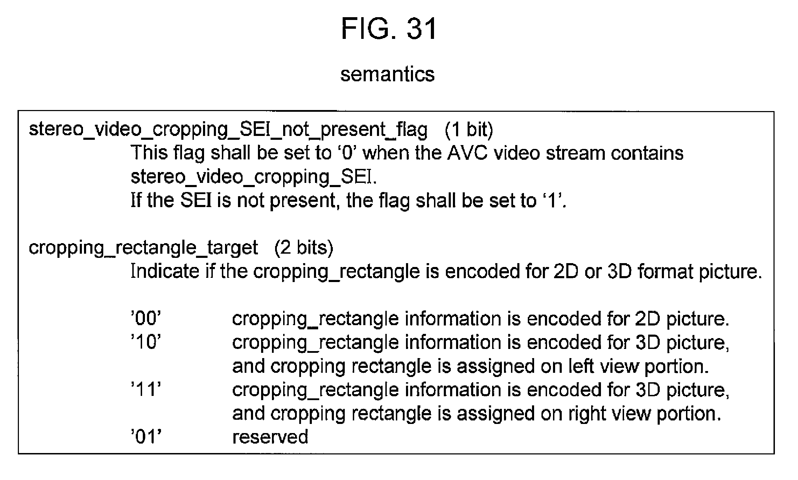

For example, identification information indicating whether cropping information is 2D or 3D image cropping information is inserted into "AVC_video_descriptor" contained in a video elementary loop (Video ES loop). Figs. 30 and 31 respectively illustrate an example of the structure (Syntax) and an example of the data definition contents (semantics) of "AVC_video_descriptor" having identification information. The descriptor itself is already contained in the H.264/AVC standards.

In this descriptor, a two-bit "cropping_rectangle_target" field is newly defined. The example of the structure of "AVC_video_descriptor" shown in Fig. 30 is obtained by adding this two-bit field to the example of the structure "AVC_video_descriptor" (see Fig. 7) of the above-described first embodiment. In the fourth embodiment, one-bit flag information "stereo_video_cropping_SEI_Not_present_flag" is not necessary. However, in the fourth embodiment, as in the first embodiment, a SEI "Stereo_Video_Cropping SEI" may be newly defined. In this case, this one-bit flag information becomes valid.

The two-bit "cropping_rectangle_target" field is identification information indicating whether cropping information is 2D or 3D image cropping information. "00" indicates that cropping information is 2D image cropping information. "01" indicates that cropping information is 3D image cropping information and that the specified rectangular area corresponds to a left-eye area. "10" indicates that cropping information is 3D image cropping information and that the specified rectangular area corresponds to a right-eye area.

Also, identification information indicating whether cropping information is 2D or 3D image cropping information is inserted as a program descriptor of the program map table. In this case, "Stereo_Video_cropping_descriptor" having this identification information is newly defined, and in this descriptor, the two-bit "cropping_rectangle_target" field is defined.

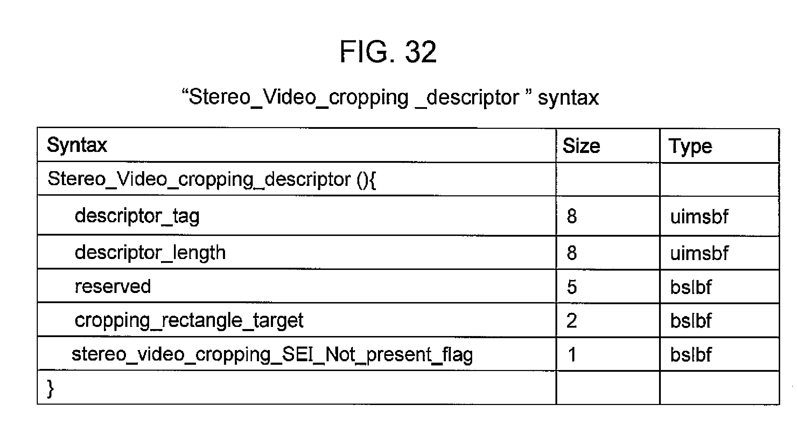

Fig. 32 illustrates an example of the structure (Syntax) of "Stereo_Video_cropping_descriptor". The eight-bit "descriptor_tag" field indicates that this descriptor is "Stereo_Video_cropping_descriptor". The eight-bit "descriptor_length" field indicates the number of bytes of the fields after the "descriptor_length" field.

The example of the structure of "Stereo_Video_cropping_descriptor" shown in Fig. 32 is obtained by adding the two-bit field "cropping_rectangle_target" to the example of the structure "Stereo_Video_cropping_descriptor" (see Fig. 9) of the above-described first embodiment. In the fourth embodiment, one-bit flag information "stereo_video_cropping_SEI_Not_present_flag" is not necessary. However, in the fourth embodiment, as in the first embodiment, a SEI "Stereo_Video_Cropping SEI" may be newly defined. In this case, this one-bit flag information becomes valid.

The two-bit "cropping_rectangle_target" field is identification information indicating whether cropping information is 2D or 3D image cropping information. "00" indicates that cropping information is 2D image cropping information. "01" indicates that cropping information is 3D image cropping information and that the specified rectangular area corresponds to a left-eye area. "10" indicates that cropping information is 3D image cropping information and that the specified rectangular area corresponds to a right-eye area.

### <5. Fifth Embodiment>

### "Image Transmitting/Receiving System"

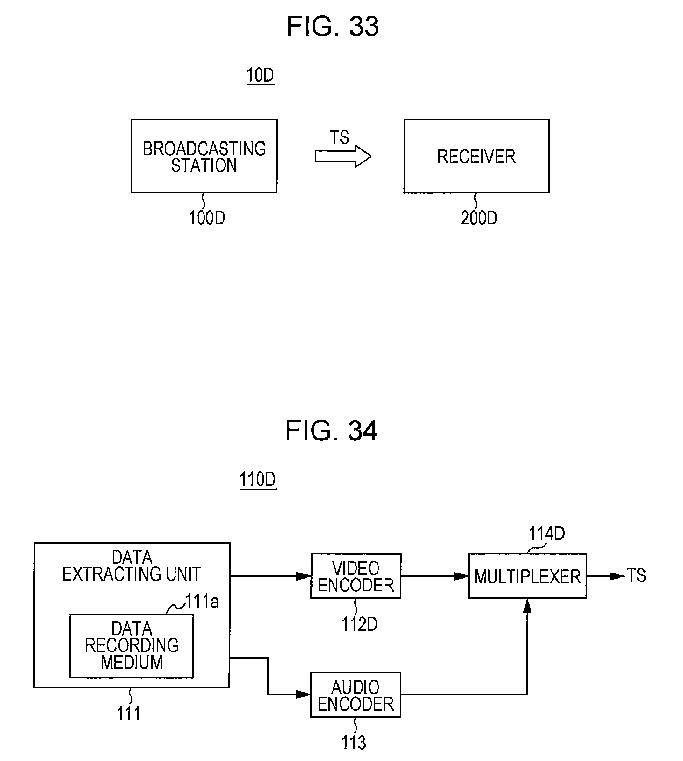

Fig. 33 illustrates an example of the configuration of an image transmitting/receiving system 10D in accordance with a fifth embodiment. The image transmitting/receiving system 10D includes a broadcasting station 100D and a receiver 200D. The broadcasting station 100D transmits through broadcasting waves a transport stream (multiplexed data stream data) TS including a video data stream containing three-dimensional (3D) image data having left-eye image data and right-eye image data. The transmission format of this three-dimensional image data may be a side-by-side mode (see part (a) of Fig. 39) or a top-and-bottom mode (see part (b) of Fig. 39).

In this fifth embodiment, it is assumed that three-dimensional image data has a 1920 x 1080-pixel format. The broadcasting station 100D encodes the 3D image data in units of 16x16 blocks. Accordingly, the broadcasting station 100D adds eight lines formed of blank data to the three-dimensional image data, making the image data be 1920-pixel x 1088-line image data, which is then encoded. Three-dimensional (3D) display cropping information is inserted into the header of a video data stream. Also, transmission format information concerning the transmission format of the three-dimensional image data is inserted into the header of the video data stream.

The receiver 200D receives a transport stream TS transmitted through broadcasting waves from the broadcasting station 100D. The receiver 200D obtains side-by-side mode (see part (a) of Fig. 39) or top-and-bottom mode (see part (b) of Fig. 39) three-dimensional image data including left-eye image data and right-eye image data from the received transport stream TS.

As described above, in the broadcasting station 100D, in order to perform encoding in units of 16x16 blocks, eight lines formed of blank data have been added to 1920-pixel x 1080-line image data, making the image data be 1920-pixel x 1088-line image data, which is then encoded. Accordingly, the receiver 200D obtains 1920-pixel x 1088-line image data including eight lines formed of blank data as the three-dimensional image data after decoding.

If the receiver 200D is a television receiver (2DTV) which does not support 3D display, i.e., a receiver which can perform only 2D display, it performs image data cropping processing and scaling processing on the basis of the 3D display cropping information and the transmission format information inserted into the header of the video data stream. More specifically, the receiver 200D crops part of three-dimensional image data, for example, left-eye image data, on the basis of this cropping information and the transmission format information, and then, performs scaling processing on the cropped image data in the direction corresponding to the transmission format, thereby generating 2DTV display image data.

For example, if the side-by-side mode is employed, the receiver 200D crops 960-pixel x 1080-line left-eye image data from the 1920-pixel x 1080-line image data, which contains actual image data. Then, the receiver 200D performs horizontal scaling processing on this left-eye image data so as to generate 2DTV 1920-pixel x 1080-line display image data.

Alternatively, if the top-and-bottom mode is employed, the receiver 200D crops 1920-pixel x 540-line left-eye image data from the 1920-pixel x 1080-line image data, which contains actual image data. Then, the receiver 200D performs vertical scaling processing on this left-eye image data so as to generate 2DTV 1920-pixel x 1080-line display image data.

On the other hand, if the receiver 200D is a television receiver (3DTV) which can perform 3D display, it crops 1920-pixel x 1080-line image data, which contains actual image data, from the 3D image data on the basis of the 3D display cropping information inserted into the header of the video data stream, thereby generating 3DTV left-eye display image data and right-eye display image data.

For example, if the side-by-side mode is employed, the receiver 200D crops the 1920-pixel x 1080-line image data, which contains actual image data. The receiver 200D then divides this image data into a left frame and a right frame and performs scaling processing on each of the left and right frames, thereby generating 3DTV 1920-pixel x 1080-line left-eye display image data and right-eye display image data.

Alternatively, if the top-and-bottom mode is employed, the receiver 200D crops the 1920-pixel x 1080-line image data, which contains actual image data. The receiver 200D then divides this image data into a top frame and a bottom frame and performs scaling processing on each of the top and bottom frames, thereby generating 3DTV 1920-pixel x 1080-line left-eye display image data and right-eye display image data.

### "Example of Configuration of Transmission Data Generator"

Fig. 34 illustrates an example of the configuration of a transmission data generator 110D for generating the above-described transport stream TS in the broadcasting station 100D. The transmission data generator 110D includes a data extracting unit (archive) 111, a video encoder 112D, an audio encoder 113, and a multiplexer 114D. In Fig. 34, elements corresponding to those shown in Fig. 2 are designated by like reference numerals, and a detailed explanation thereof is omitted as appropriate.

The video encoder 112D encodes three-dimensional image data output from the data extracting unit 111 by using H.264/AVC (Advanced Video Coding) so as to obtain encoded video data. The video encoder 112D also generates a video elementary stream (video data stream) including the encoded video data by using a stream formatter (not shown), which is provided subsequent to the video encoder 112D.

In this case, the video encoder 112D inserts 3D display cropping information (see Fig. 4) into the header of the video data stream. 3D display cropping information is inserted into a SPS (Sequence Parameter Set) of an access unit (see Fig. 16). "Frame Packing Arrangement SEI message" is inserted into SEIs of the access unit (see Fig. 16). In this SEI, type information (transmission format information) indicating what type of transmission format is used for the three-dimensional image data is contained.

The multiplexer 114D packetizes the elementary streams generated by the video encoder 112D and the audio encoder 113, and multiplexes the packetized streams so as to generate a transport stream (multiplexed data stream) TS.

The other components of the transmission data generator 110D shown in Fig. 34 are configured and operated similarly to those of the transmission data generator 110 shown in Fig. 2. In the transmission data generator 110D shown in Fig. 34, the multiplexer 114D generates the following transport stream (multiplexed data stream) TS. The transport stream TS includes a video data stream containing three-dimensional image data having left-eye image data and right-eye image data. 3D display cropping information is inserted into the header of the video data stream.

### "Example of Configuration of Receiver"

The receiver 200D, which is a television receiver (3DTV) that can perform 3D display, is not shown, and is configured and operated similarly to that of the receiver 200 shown in Fig. 10. The 3D signal processor 216 crops, on the basis of the 3D display cropping information inserted into the SPS (Sequence Parameter Set) of an access unit, 1920-pixel x 1080-line image data, which contains actual image data, so as to generate 3DTV left-eye display image data SL and right-eye display image data SR.

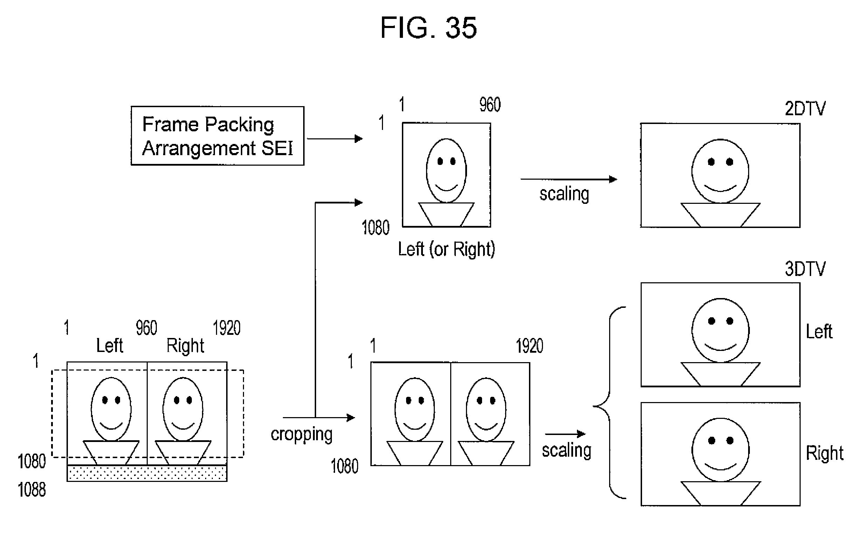

More specifically, if the side-by-side mode is employed, as shown in Fig. 35, the 3D signal processor 216D crops the 1920-pixel x 1088-line image data, which contains actual image data, from the 1920-pixel x 1080-line image data. The 3D signal processor 216D then divides this image data into left and right frames and performs horizontal scaling processing on each of the left and right frames, thereby generating 3DTV 1920-pixel x 1080-line left-eye display image data SL and right-eye display image data SR.

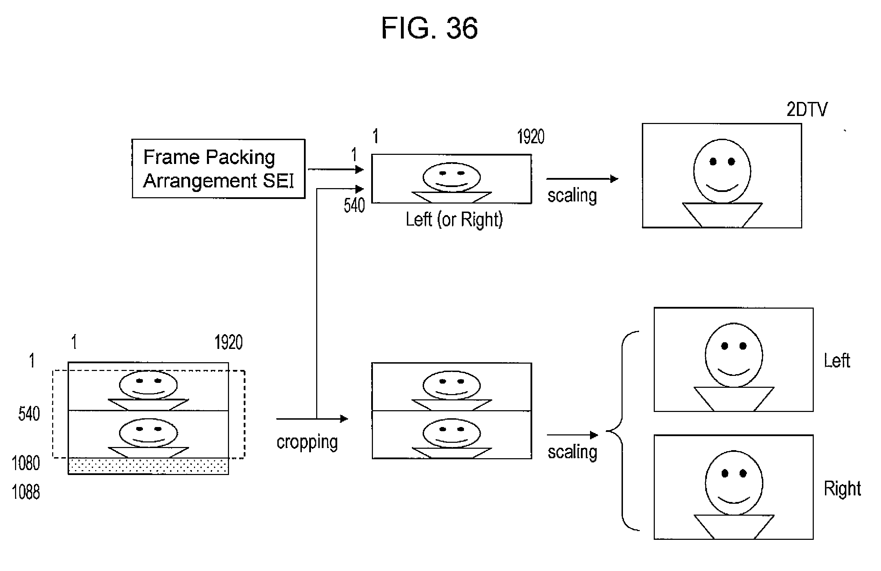

Alternatively, if the top-and-bottom mode is employed, as shown in Fig. 36, the 3D signal processor 216D crops the 1920-pixel x 1088-line image data, which contains actual image data, from the 1920-pixel x 1080-line image data. The 3D signal processor 216D then divides this image data into top and bottom frames and performs vertical scaling processing on each of the top and bottom frames, thereby generating 3DTV 1920-pixel x 1080-line left-eye display image data SL and right-eye display image data SR.

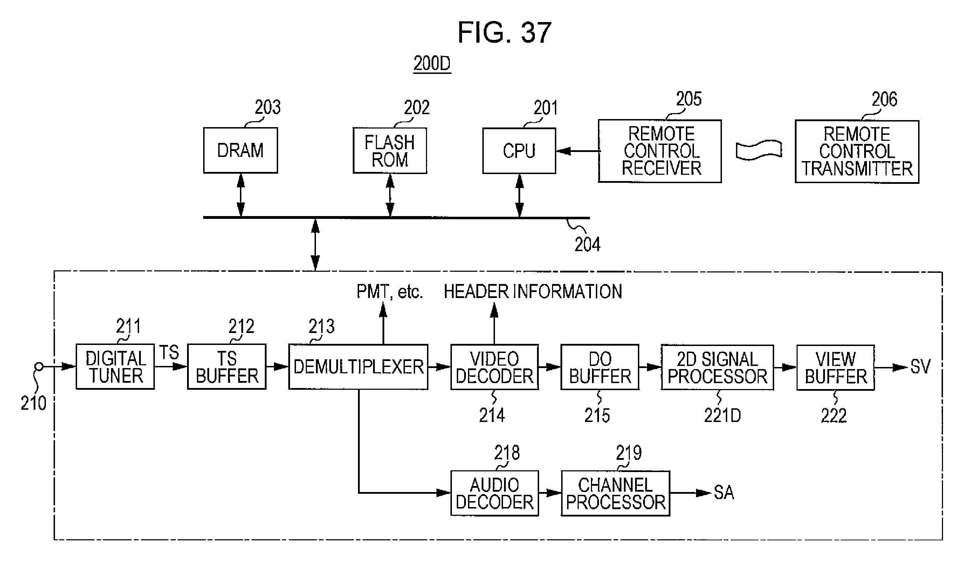

Fig. 37 illustrates an example of the configuration of the receiver 200D, which is a television receiver (2DTV) that performs 2D display. In Fig. 37, elements corresponding to those shown in Fig. 13 are designated by like reference numerals, and a detailed explanation thereof is omitted as appropriate.

The video decoder 214 decodes the encoded image data contained in the video elementary stream extracted by the demultiplexer 213 so as to obtain decoded three-dimensional image data. This three-dimensional image data is 1920-pixel x 1088-line image data including eight-line blank data. This three-dimensional image data is then temporarily stored in the DO buffer 215.

The video decoder 214 also extracts header information of the video data stream and supplies the header information to the CPU 201. In this case, 3D display cropping information and transmission format information for three-dimensional image data are contained in the SPS of an access unit. Image data cropping processing performed by a 2D signal processor 221D, which will be discussed later, is controlled on the basis of the cropping information and the transmission format information.

The CPU 201 converts the 3D display cropping information into 2D display cropping information. For example, if the transmission format of the three-dimensional image data is the side-by-side mode, the value "frame_crop_right_offset" indicating the horizontal end position, i.e., the right edge position, is reduced by 1/2. If the transmission format of the three-dimensional image data is the top-and-bottom mode, the value "frame_crop_bottom_offset" indicating the vertical end position, i.e., the bottom edge position, is reduced by 1/2.

The 2D signal processor 221D crops, for example, left-eye image data, on the basis of the 2D display cropping information, from the 3D image data stored in the DO buffer 215 so as to generate 2DTV display image data SV.

If, for example, the side-by-side mode is employed, as shown in Fig. 35, the 2D signal processor 221D crops, for example, 960-pixel x 1080-line left-eye image data, from 1920-pixel x 1080-line image data, which contains actual image data. Then, the 2D signal processor 221D performs scaling processing on this left-eye image data so as to generate 2DTV 1920-pixel x 1080-line display image data SV.

If, for example, the top-and-bottom mode is employed, as shown in Fig. 36, the 2D signal processor 221D crops, for example, 1920-pixel x 540-line left-eye image data, from 1920-pixel x 1080-line image data, which contains actual image data. Then, the 2D signal processor 221 performs scaling processing on the left-eye image data so as to generate 2DTV 1920-pixel x 1080-line display image data SV.

The other components of the receiver 200D shown in Fig. 37 are configured and operated as those of the receiver 200a shown in Fig. 13. In the receiver 200D shown in Fig. 37, the 2D signal processor 221D performs image data cropping and scaling on the basis of the 3D display cropping information and the transmission format information for three-dimensional image data inserted into the header of the video stream data so as to correctly generate two-dimensional display image data, thereby performing correct 2D display.

### Industrial Applicability

This invention is applicable to, for example, an image transmitting/receiving system that transmits side-by-side or top-and-bottom three-dimensional image data through broadcasting waves.

### Reference Signs List

10, 10A to 10D ... image transmitting/receiving system
100, 100A to 100D ... broadcasting station
110, 110A, 110C, 110D ... transmission data generator
111 ... data extracting unit
111a ... data recording medium
112, 112A, 112C, 112D ... video encoder
113 ... audio encoder
114, 114A, 114C, 114D ... multiplexer
200, 200A to 200C ... receiver
201 ... CPU
202 ... flash ROM
203 ... DRAM
204 ... internal bus
205 ... remote control receiver
206 ... remote control transmitter
210 ... antenna terminal
211 ... digital tuner
212 ... transport stream buffer (TS buffer)
213 ... demultiplexer
214 ... video decoder
215 ... display output buffer (DO buffer)
216, 216A to 216C ... 3D signal processor
217L, 217R ... view buffer
218 ... audio decoder
219 ... channel processor
221, 221D ... 2D signal processor
222 ... view buffer

## Claims

1. An image data transmitting apparatus comprising:
an image data output unit that outputs three-dimensional image data including left-eye image data and right-eye image data; and
a transmitter that transmits a multiplexed data stream including a data stream, the data stream including the three-dimensional image data output from the image data output unit, first cropping information used for two-dimensional display and second cropping information used for three-dimensional display being inserted into a header of the data stream.

2. The image data transmitting apparatus according to Claim 1, wherein the transmitter inserts, into a higher layer of the data stream, flag information indicating whether the second cropping information is present in the header of the data stream.

3. The image data transmitting apparatus according to Claim 2, wherein:
the multiplexed data stream includes a program map table, which serves as program specific information indicating to which program each elementary stream contained in the multiplexed data stream belongs; and
the transmitter inserts the flag information under the program map table.

4. The image data transmitting apparatus according to Claim 3, wherein the transmitter inserts the flag information as a program descriptor of the program map table.

5. The image data transmitting apparatus according to Claim 3, wherein the transmitter inserts the flag information under a video elementary loop of the program map table.

6. The image data transmitting apparatus according to Claim 1, wherein information indicating until when a cropping state represented by the second cropping information continues is added to the second cropping information.

7. An image data transmitting method comprising:
an image data output step of outputting three-dimensional image data including left-eye image data and right-eye image data; and
a transmitting step of transmitting a multiplexed data stream including a data stream, the data stream including the three-dimensional image data which is output in the image data output step, first cropping information used for two-dimensional display and second cropping information used for three-dimensional display being inserted into a header of the data stream.

8. An image data receiving apparatus comprising:
a receiver that receives a multiplexed data stream including a data stream, the data stream including three-dimensional image data having left-eye image data and right-eye image data, first cropping information used for two-dimensional display and second cropping information used for three-dimensional display being inserted into a header of the data stream; and
an image data processor that generates left-eye and right-eye display image data on the basis of the three-dimensional image data obtained from the multiplexed data stream received by the receiver,
wherein the image data processor performs image data cropping processing on the basis of the second cropping information contained in the header of the data stream.

9. An image data receiving method comprising:
a receiving step of receiving a multiplexed data stream including a data stream, the data stream including three-dimensional image data having left-eye image data and right-eye image data, first cropping information used for two-dimensional display and second cropping information used for three-dimensional display being inserted into a header of the data stream; and
an image data processing step of generating left-eye and right-eye display image data on the basis of the three-dimensional image data obtained from the multiplexed data stream received in the receiving step,
wherein, in the image data processing step, image data cropping processing is performed on the basis of the second cropping information contained in the header of the data stream.

10. An image data receiving apparatus comprising:
a receiver that receives a multiplexed data stream including a data stream, the data stream including a three-dimensional image data having left-eye image data and right-eye image data, cropping information used for two-dimensional display being inserted into a header of the data stream; and
an image data processor that generates left-eye and right-eye display image data on the basis of the three-dimensional image data obtained from the multiplexed data stream received by the receiver,
wherein the image data processor converts the cropping information used for two-dimensional display contained in the header of the data stream into cropping information used for three-dimensional display and performs image data cropping processing from the three-dimensional image data obtained from the multiplexed data stream received by the receiver on the basis of the cropping information used for three-dimensional display.

11. An image data receiving method comprising:
a receiving step of receiving a multiplexed data stream including a data stream, the data stream including three-dimensional image data having left-eye image data and right-eye image data, cropping information used for two-dimensional display being inserted into a header of the data stream; and
an image data processing step of generating left-eye and right-eye display image data on the basis of the three-dimensional image data obtained from the multiplexed data stream received in the receiving step,
wherein, in the image data processing step, the cropping information used for two-dimensional display contained in the header of the data stream is converted into cropping information used for three-dimensional display, and image data cropping processing for cropping image data from the three-dimensional image data obtained from the multiplexed data stream received by the receiving step is performed on the basis of the cropping information used for three-dimensional display.

12. An image data receiving apparatus comprising:
a receiver that receives a multiplexed data stream including a data stream, the data stream including three-dimensional image data having left-eye image data and right-eye image data, cropping information used for two-dimensional display being inserted into a header of the data stream; and
an image data processor that generates left-eye and right-eye display image data on the basis of the three-dimensional image data obtained from the multiplexed data stream received by the receiver,
wherein the image data processor performs image data cropping processing on the basis of the cropping information used for two-dimensional display so as to generate one of left-eye and right-eye display image data, and the image data processor generates the other one of the left-eye and the right-eye display image data on the basis of image data that remains after performing the image data cropping processing on the basis of the cropping information used for two-dimensional display.

13. An image data receiving method comprising:
a receiving step of receiving a multiplexed data stream including a data stream, the data stream including three-dimensional image data having left-eye image data and right-eye image data, cropping information used for two-dimensional display being inserted into a header of the data stream; and
an image data processing step of generating left-eye and right-eye display image data on the basis of the three-dimensional image data obtained from the multiplexed data stream received in the receiving step,
wherein, in the image data processing step, image data cropping processing is performed on the basis of the cropping information used for two-dimensional display so as to generate one of left-eye and right-eye display image data, and the other one of the left-eye and the right-eye display image data is generated on the basis of image data that remains after performing the image data cropping processing on the basis of the cropping information used for two-dimensional display.

14. An image data transmitting apparatus comprising:
an image data output unit that outputs three-dimensional image data including left-eye image data and right-eye image data; and
a transmitter that transmits a multiplexed data stream including a data stream, the data stream including the three-dimensional image data output from the image data output unit, cropping information being inserted into a header of the data stream,
wherein the transmitter inserts, into the header of the data stream or a higher layer of the data stream, identification information for identifying whether the cropping information is cropping information used for a two-dimensional image or cropping information used for a three-dimensional image.

15. The image data transmitting apparatus according to Claim 14, wherein the identification information for identifying that the cropping information is cropping information used for a three-dimensional image includes information indicating whether the cropping information is cropping information for left-eye image data or cropping information for right-eye image data.

16. The image data transmitting apparatus according to Claim 14, wherein:
the identification information is inserted into the header of the data stream; and
information indicating until when a cropping state represented by the cropping information continues is added to the identification information.

17. The image data transmitting apparatus according to Claim 14, wherein:
the multiplexed data stream includes a program map table, which serves as program specific information indicating to which program each elementary stream contained in the multiplexed data stream belongs; and
the transmitter inserts the identification information under the program map table.

18. An image data transmitting method comprising:
an image data output step of outputting three-dimensional image data including left-eye image data and right-eye image data; and
a transmitting step of transmitting a multiplexed data stream including a data stream, the data stream including the three-dimensional image data which is output in the image data output step, cropping information being inserted into a header of the data stream,
wherein, in the transmitting step, identification information for identifying whether the cropping information is cropping information used for a two-dimensional image or cropping information used for a three-dimensional image is inserted into the header of the data stream or a higher layer of the data stream.

19. An image data receiving apparatus comprising:
a receiver that receives a multiplexed data stream including a data stream, the data stream including three-dimensional image data having left-eye image data and right-eye image data, cropping information used for three-dimensional display and transmission format information for the three-dimensional image data being inserted into a header of the data stream; and
an image data processor that generates two-dimensional display image data on the basis of the three-dimensional image data obtained from the multiplexed data stream received by the receiver,
wherein the image data processor performs image data cropping processing and scaling processing on the basis of the cropping information used for three-dimensional display and the transmission format information for the three-dimensional image data contained in the header of the data stream.

20. An image data receiving method comprising:
a receiving step of receiving a multiplexed data stream including a data stream, the data stream including three-dimensional image data having left-eye image data and right-eye image data, cropping information used for three-dimensional display and transmission format information for the three-dimensional image data being inserted into a header of the data stream; and
an image data processing step of generating two-dimensional display image data on the basis of the three-dimensional image data obtained from the multiplexed data stream received in the receiving step,
wherein, in the image data processing step, image data cropping processing and scaling processing are performed on the basis of the cropping information used for three-dimensional display and the transmission format information for the three-dimensional image data contained in the header of the data stream.
